# FASCICULE DE BREVET EUROPEEN

(11) **EP 0 353 123 B1**
(45) Date de publication et mention de la délivrance du brevet: **24.08.1994**
(21) Numéro de dépôt: 89402001.5
(22) Date de dépôt: 12.07.1989
(51) Int. Cl.: C07D 239/50, C07D 498/04, A61K 7/06

(54) **Nouveaux dérivés d'uréylène triamino-2,4,6 pyrimidine oxyde-3, leur préparation et leur utilisation en cosmétique et dermopharmacie**
Harnstoffhaltige 2,4,6-Triaminopyrimidin-3-oxid-Derivate, deren Herstellung und deren Verwendung für Kosmetika und Hautpharmazie
Ureic 2,4,6-triamino-pyrimidine-3-oxide derivatives, their preparation and their cosmetical and dermapharmaceutical use

(30) Priorité: 29.07.1988 LU 87304
(43) Date de publication de la demande: 31.01.1990
(73) Titulaire: L'OREAL, 75008 Paris (FR)
(72) Inventeur: Maignan, Jean, F-93290 Tremblay-les-Gonesse (FR); Lang, Gérard, F-95210 Saint-Gratien (FR)
(74) Mandataire: Casalonga, Axel

(56) Documents cités:
- EP-A- 0 042 669
- EP-A- 0 303 871
- EP-A- 0 304 649
- EP-A- 0 304 665
- WO-A-86/00616
- US-A- 4 139 619

## Description

La présente invention est relative à de nouveaux dérivés de pyrimidine oxyde-3, leur préparation et à des compositions cosmétiques ou pharmaceutiques destinées notamment à être utilisées en application topique.

On connaît déjà, dans l'état de la technique, la pipéridino-6 diamino-2,4 pyrimidine oxyde-3 ou "Minoxidil" pour ses propriétés d'agent antihypertenseur, mais également pour son utilisation dans le traitement de la chute des cheveux, de la pelade, de la dermatite desquamante, de l'alopécie, etc.

On connait également d'autres dérivés de la triamino-2,4,6 pyrimidine oxyde-3 non substitués en positions 2 et 4 utilisés comme agent stimulant la croissance des cheveux. De tels composés avec le "minoxidil" ont été décrits dans le brevet US 4 139 619 (UPJOHN).

Des dérivés mono ou dicarbamates dans les positions 2 et/ou 4 du triamino-2,4,6 pyrimidine oxyde-3 ont été utilisés d'après la demande WO/86/00616 (BAZZANO) dans le traitement de la chute des cheveux et notamment de certains types d 'alopécie.

La demanderesse avait découvert de nouveaux dérivés de pyrimidine oxyde-3 qui sont des mono- ou des diurées dans les positions 2 et/ou 4 du triamino-2,4,6 pyrimidine oxyde-3.

Ces composés ont fait l'objet d'une demande de brevet au Luxembourg n° 86 959.

Les composés monourées étaient synthétisés par addition d'un isocyanate ou par réaction d'un chlorure de carbamoyle sur l'une des deux fonctions amine des triamino-2,4,6 pyrimidine oxyde-3.

Les composés diurées étaient synthétisés en utilisant un excès d'isocyanate.

Dans les deux cas précités, à partir des isocyanates ou des chlorures de carbamoyle, les urées obtenues ne pouvaient pas comporter des groupements hydrophiles tels que des radicaux hydroxyle ou amine; ces radicaux étant incompatibles avec une fonction isocyanate ou chlorure de carbamoyle. De plus, le nombre de chlorure de carbamoyle commerciaux est très limité. Ainsi, les chlorures de carbamoyle dans lesquels l'atome d'azote fait partie d'un cycle ne sont pas commercialisés.

La demanderesse vient de découvrir de nouveaux produits dérivés de la pyrimidine oxyde-3 qui sont des mono-urées dans les positions 2 ou 4 du triamino-2,4,6 pyrimidine oxyde-3 dont l'atome d'azote de la fonction urée qui n'est pas relié au noyau pyrimidine est substitué ou disubstitué par un radical comportant une ou plusieurs fonction(s) hydroxyle ou amine ou forme lorsqu'il est disubstitué un hétérocycle comportant un ou plusieurs hétéroatome (s).

Elle a découvert que ces nouvelles urées étaient particulièrement efficaces pour la repousse des cheveux et pouvaient être utilisées notamment dans le traitement des maladies du cuir chevelu, telles que la pelade, la chute des cheveux, la dermatite desquamante, l'alopécie.

L'invention a donc pour objet de nouveaux dérivés de triamino-2,4,6 pyrimidine oxyde-3.

Un autre objet de l'invention est constitué par leur procédé de préparation.

L'invention concerne également les compositions cosmétiques et/ou pharmaceutiques mettant en oeuvre ces composés.

D'autres objets de l'invention apparaîtront à la lecture de la description et des exemples qui suivent.

Les composés conformes à l'invention sont essentiellement caractérisés par le fait qu'ils répondent à la formule : 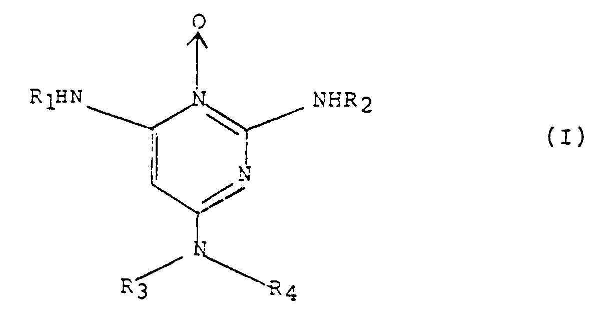
dans laquelle :
R₃ et R₄, identiques ou différents, désignent un atome d'hydrogène, un radical alkyle, linéaire ou ramifié en C₁-C₁₈, alcényle ayant 2 à 18 atomes de carbone, cycloalkyle ayant de 5 à 8 atomes de carbone, éventuellement substitués par un ou plusieurs groupement(s) alkyle inférieur, un groupement cycloalcène, les groupements alkyle, alcényle ou cycloalkyle pouvant être eux-mêmes substitués par un ou plusieurs groupement(s) hydroxyle,
R₃ et/ou R₄ désignent également un groupement aryle ou aralkyle, répondant à la formule : 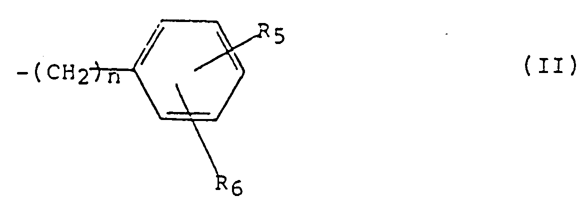
dans laquelle :
n peut prendre les valeurs de 0 à 4 et où R₅ et/ou R₆, indépendamment l'un de l'autre, désignent hydrogène, un groupement alkyle inférieur en C₁-C₆, un groupement nitro, hydroxyle, alcoxy ou un atome d'halogène, ou encore un groupement carboxylique, ainsi que les formes salifiées, esters et amides de ces derniers,
R₃ et R₄, pris ensemble avec l'atome d'azote auquel ils sont reliés, peuvent former un hétérocycle de 3 à 7 atomes de carbone,
R₁ et R₂,
désignent un atome d'hydrogène ou un groupement carbamoyle, de formule : 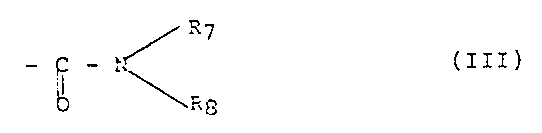
sous réserve que l'un désigne l'hydrogène et l'autre un groupement carbamoyle ;
et dans laquelle :
R₇ et/ou R₈ désignent, indépendamment l'un de l'autre, un atome d'hydrogène, un groupement alkyle, linéaire ou ramifié en C₁-C₁₈, substitué par un ou plusieurs groupement(s) hydroxyle et/ou amino, cette chaîne alkyle pouvant éventuellement être interrompue par un ou plusieurs hétéroatome(s) tel(s) qu'un atome de soufre, d'azote ou d'oxygène, un groupement cycloalkyle, aryle ou aralkyle, substitué par un groupement hydroxyle, amino et/ou carboxylique ; R₇ et/ou R₈ peuvent désigner également un reste de sucre ; R₇ et R₈ peuvent former avec l'atome d'azote auquel ils sont rattachés, un hétérocycle comportant un ou plusieurs hétéroatome(s).

Les composés conformes à l'invention peuvent également exister sous la forme tautomère répondant aux formules (IA) et (IB) suivantes : 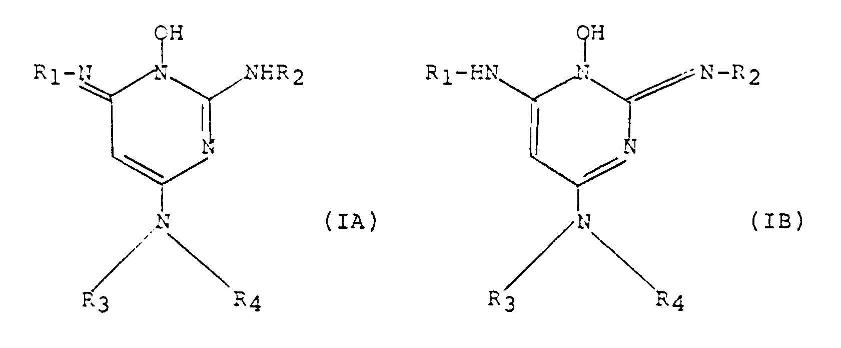
Il est entendu que ces formes tautomères (I), (IA) et (IB) peuvent être présentées dans le mélange dans des proportions variables et l'une peut être prépondérante par rapport aux autres suivant la nature des substituants R₃, R₄, R₁, R₂ ou suivant la nature du solvant.

Conformément à l'invention, les radicaux alkyle ayant de 1 à 18 atomes de carbone, substitués par un ou plusieurs groupement(s) hydroxyle(s) ou amino, sont de préférence choisis parmi les radicaux hydroxy-2 éthyle, dihydroxy-2,3 propyle, hydroxy-2 propyle, hydroxy-2 éthoxyéthyle, hydroxy-2 éthylaminoéthyle, amino-2 éthyle.

Les radicaux cycloalkyle, subtitués par un ou plusieurs groupement(s) hydroxyle(s) ou amino sont de préférence des groupements cycloalkyle en C₅-C₈. Lorsque R₇ et R₈ forment avec l'atome d'azote auquel ils sont rattachés un hétérocycle comportant un ou plusieurs hétéroatome(s), on entend de préférence les groupements morpholino, pipéridino, pyrrolidino, pipérazino, N-alkyl 4′-pipérazino, dans lequel le groupement alkyle en position 4′ contient de préférence 1 à 6 atomes de carbone, dont l'un est éventuellement substitué par un groupement hydroxyle ou correspond à une thiazolidine.

Les composés préférés, conformes à l'invention, sont ceux dans lesquels l'un des groupements R₁ ou R₂ désigne un atome hydrogène, l'autre groupement R₂ ou R₁ étant différent d'hydrogène et répondent à la formule (Ia) suivante : 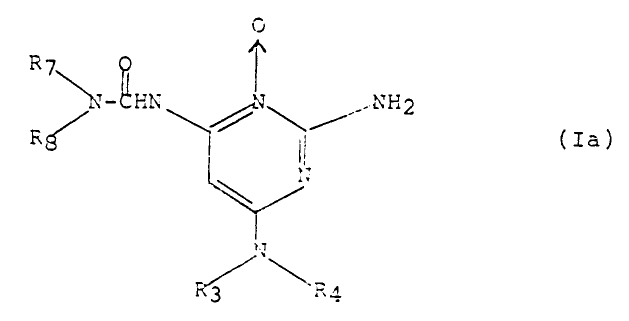
dans laquelle R₃, R₄, R₇ et R₈ ont les significations définies dans la formule (I) ci-dessus.

Les composés particulièrement préférés conformes à l'invention sont les composés de formule (Ib) et leurs isomères de formule (Ic) : 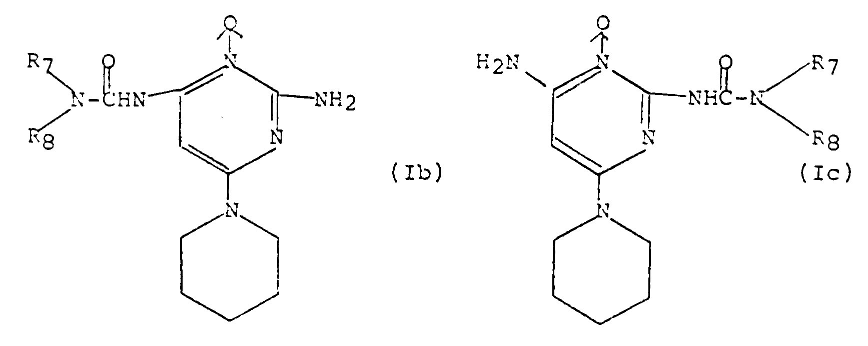
dans laquelle R₇ et R₈ ont les significations indiquées ci-dessus.

Les composés conformes à l'invention et en particulier les composés de formule (Ia) définis ci-dessus sont préparés selon une première variante, suivant un procédé consistant essentiellement à traiter l'amino-7 chloro-5 oxadiazolo-1,2,4 [2,3c] pyrimidinone-2 de formule (IVa) ou son isomère de formule (IVb) : 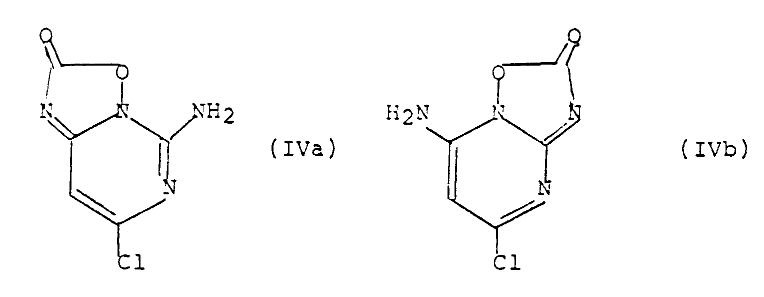
par une amine de formule : 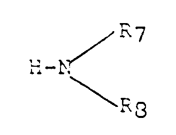
dans laquelle R₇ et R₈ ont les significations indiquées ci-dessus, puis à traiter le dérivé en résultant par une amine de formule : 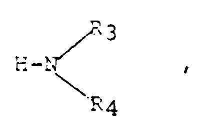
dans laquelle R₃ et R₄ ont les significations définies ci-dessus.

Le procédé conforme à l'invention peut être illustré par le schéma réactionnel A suivant. 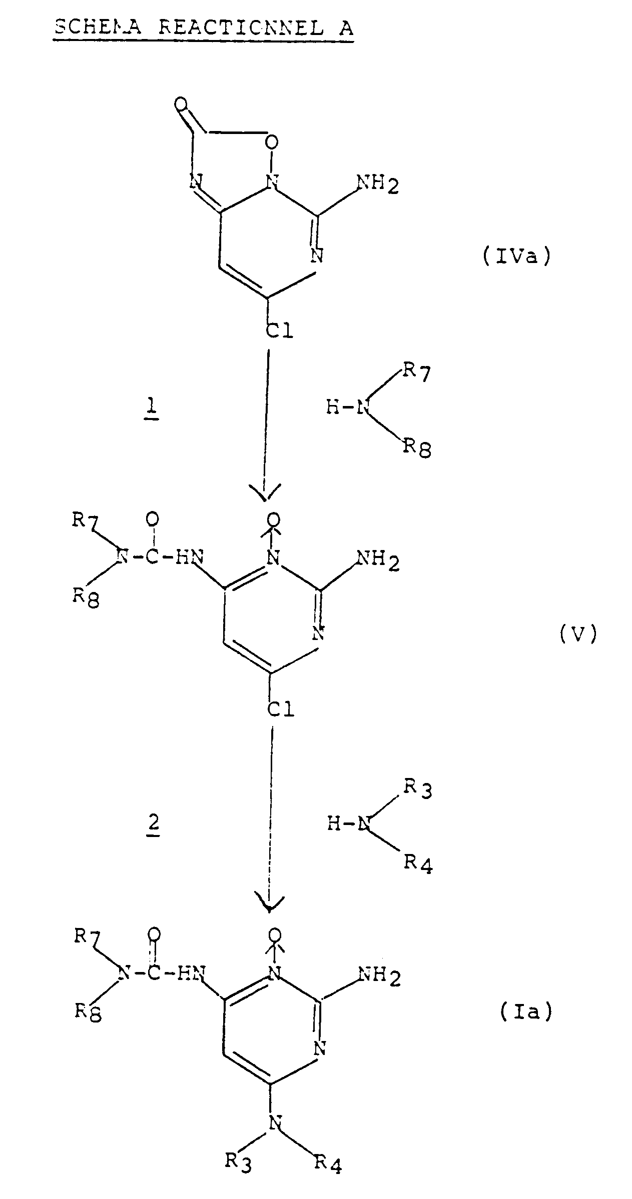
La première étape de ce procédé consiste à traiter l'amino-7 chloro-5-2H-oxadiazolo-1,2,4 [2,3c] pyrimidinone-2 par une amine de formule : 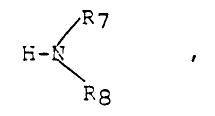
dans laquelle R₇ et R₈ ont les significations définies dans la formule (I) et correspond à l'ouverture du cycle oxadiazolone. On obtient le dérivé de monourée de formule (V) dont la fonction amino dans la position 4 est transformée en urée. 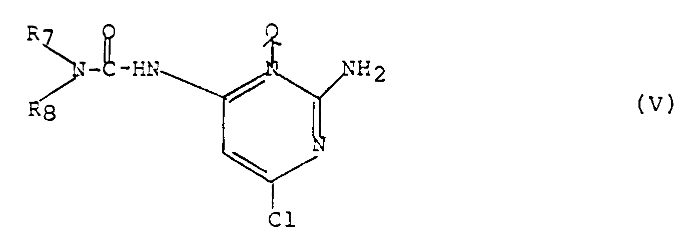
La deuxième étape consiste à faire réagir l'urée de formule (V) sur une amine de formule : 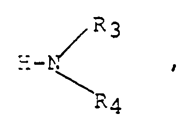
pour obtenir les composés conformes à l'invention et plus particulièrement les composés monourées de formule (Ia).

Suivant la basicité et le nombre d'équivalents de l'amine utilisée, il est possible d'obtenir directement les composés de formule (Ia) pour lesquels les groupements amino 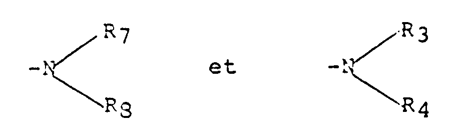
sont identiques en utilisant un excès d'amine.

Suivant la nature du groupement 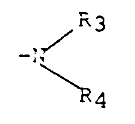
souhaité, il est préférable de procéder en deux étapes et de faire réagir l'urée répondant à la formule (V) avec l'amine correspondante.

Ce procédé est particulièrement intéressant dans le cas où le groupement amino 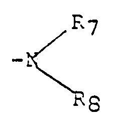
est très polaire et le groupement amino 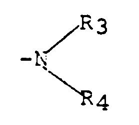
désigne un groupement morpholino, pipérazino, N-alkyl-4′ pipérazino, N,N-diéthylamino, pipéridino ou pyrrolidino.

Selon une deuxième variante, on synthétise les composés conformes à l'invention, répondant à la formule (Ib), comme illustré sur le schéma B suivant, avec un rendement quantitatif, en traitant l'amino-7 pipéridino-5-2H-oxadiazolo-1,2,4 [2,3c] pyrimidinone-2 de formule (VIa) : 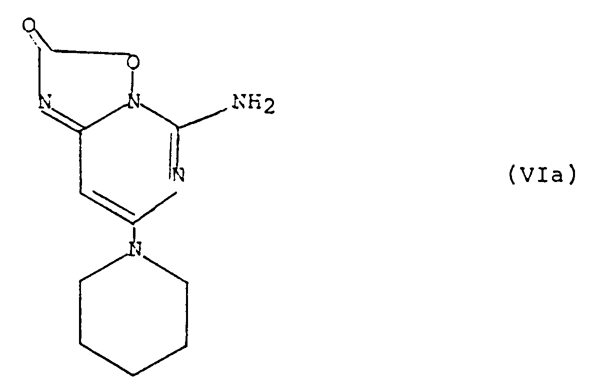
par un équivalent d'amine de formule 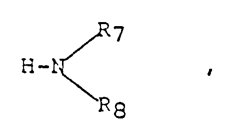
dans laquelle R₇ et R₈ ont les significations indiquées ci-dessus, comme illustré dans le schéma réactionnel B suivant : 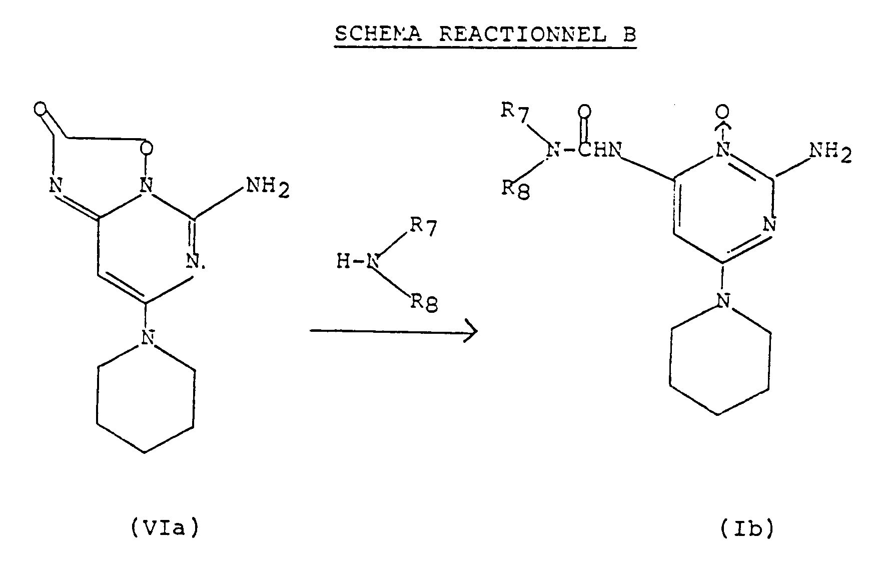
Les composés isomères répondant à la formule (Ic) conformes à l'invention, peuvent être obtenus en traitant l'amino-7 pipéridino-5-2H-oxadiazolo-1,2,4 [2,3a] pyrimidinone-2 de formule (VIb) par l'amine de formule 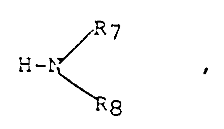
comme illustré dans le schéma réactionnel C suivant : 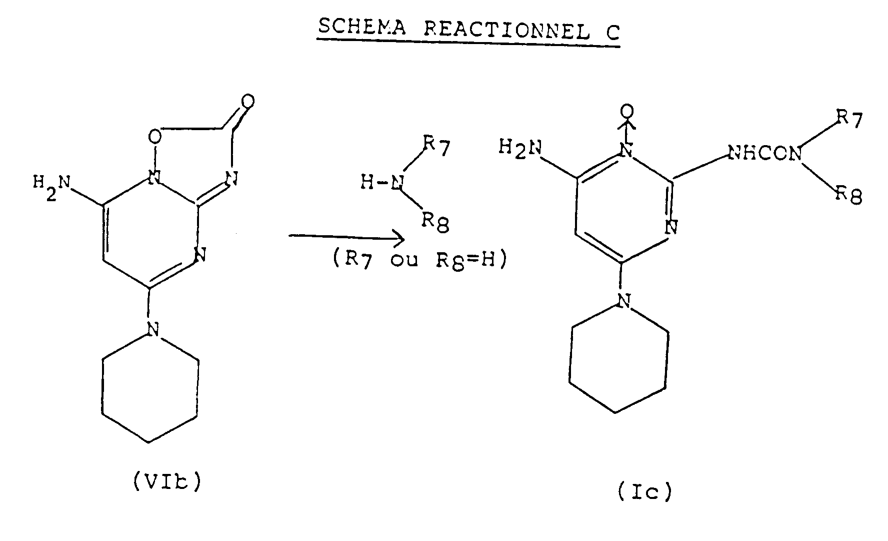
Cette synthèse est plus particulièrement appropriée lorsqu'on utilise une amine primaire (R₇ ou R₈ désignant un atome d'hydrogène).

En effet, lorsqu'on utilise une amine secondaire telle que la pipéridine, on obtient l'urée (Ic), mais celle-ci, instable, évolue vers son isomère (Ib).

La présente invention a également pour objet de nouveaux dérivés d'oxadiazolo-1,2,4 [2,3c] pyrimidinone-2 et leurs isomères, caractérisés par le fait qu'ils répondent aux formules (IVa) et (IVb) suivantes : 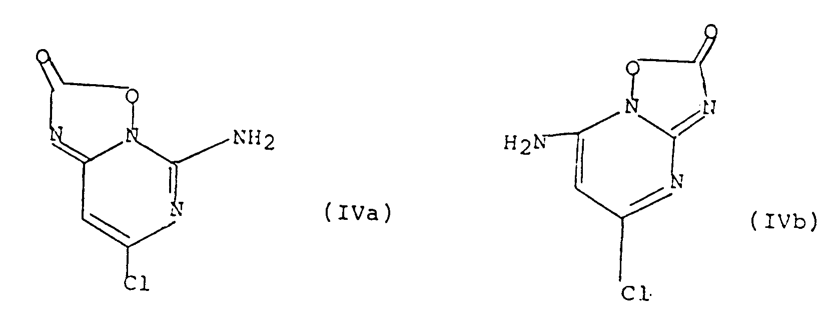
Ces dérivés constituent des composés intermédiaires utilisés pour la préparation des composés de formule (I) conformes à l'invention.

Les composés intermédiaires de formules (IVa) et (IVb), conformes à l'invention, sont préparés suivant un procédé consistant à traiter dans une première étape, la diamino-2,4 chloro-6 oxyde-3 pyrimidine de formule (VII) : 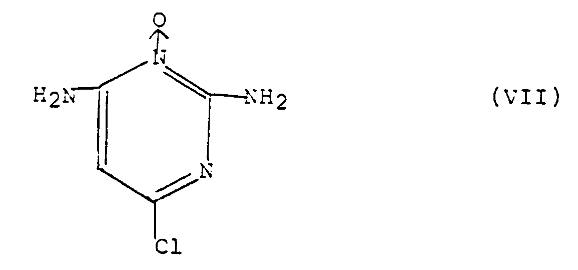
par un chlorure de carbamoyle de formule : 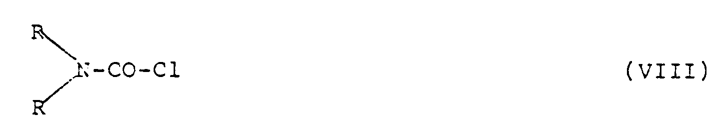
dans laquelle R désigne un groupement alkyle, dans un solvant polaire aprotique pour obtenir le mélange de dérivés monourées isomères, de formules (IXa) et (IXb) : 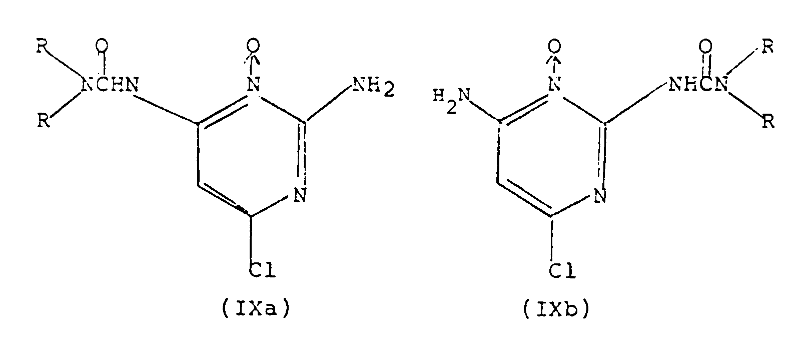
Dans une deuxième étape, on traite le mélange de dérivés monourées (IXa) et (IXb) par un excès de chlorure de carbamoyle de formule (VIII) pour obtenir l'amino-7 chloro-5-2H-oxadiazolo-1,2,4 [2,3c] pyrimidinone-2 de formule (IVa) et son isomère de formule (IVb).

Ce procédé peut être représenté suivant le schéma réactionnel D suivant : 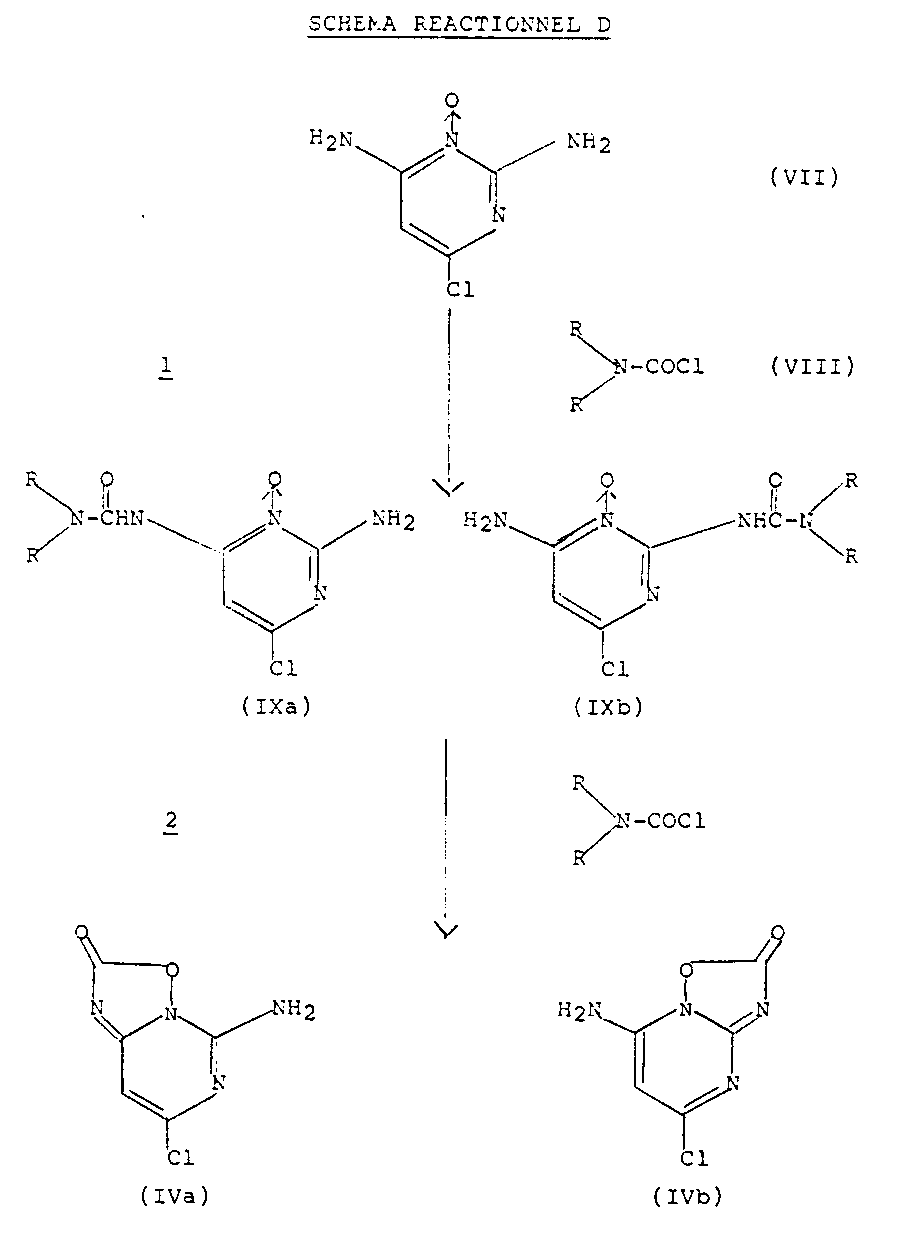
Au cours de la première étape, on utilise de préférence le chlorure de N,N-diméthylcarbamoyle. La réaction est mise en oeuvre dans un solvant polaire aprotique tel que le diméthylsulfoxyde, le tétrahydrofuranne ou le dioxane.

Le mélange des composés monourées de formules (IXa) et (IXb) est obtenu dans des proportions variant suivant le solvant utilisé.

Ainsi, en utilisant le dioxane comme solvant, le composé de formule (IXb) est majoritaire.

Il n'est pas nécessaire de purifier ce mélange pour mettre en oeuvre la deuxième étape de ce procédé.

Lorsque ce dernier est traité dans un excès de chlorure de carbamoyle de formule (VIII), le mélange constitué de l'oxadiazolo-1,2,4 [2,3c] pyrimidinone-2 et de l'oxadiazolo-1,2,4 [2,3a] pyrimidinone-2 est riche en oxadiazolo-1,2,4 [2,3c] pyrimidinone-2.

Il est donc possible d'obtenir selon ce procédé avec un bon rendement, l'amino-7 chloro-5-2H-oxadiazolo-1,2,4 [2,3c] pyrimidinone-2 de formule (IVa) pur.

Les composés conformes à l'invention peuvent être utilisés dans le domaine cosmétique ou pharmaceutique, notamment dans les applications topiques.

La demanderesse a constaté d'une façon surprenante que les composés conformes à l'invention présentent un effet cardiovasculaire nettement inférieur à celui des composés dont les fonctions amino en position 2 et/ou 4 sont libres. Ils présentent par ailleurs une bonne stabilité.

Les compositions cosmétiques ou pharmaceutiques qui constituent un autre objet de l'invention sont utilisées, notamment pour traiter le cuir chevelu et plus particulièrement la pelade, la chute des cheveux, l'alopécie, la dermatite desquamante, etc.

Ces compositions sont essentiellement caractérisées par le fait qu'elles contiennent dans un support pharmaceutiquement ou cosmétiquement approprié pour une application topique, au moins un composé répondant à la formule (I) ou aux formes tautomères de celui-ci et/ou un de ses sels, esters ou amides.

Les compositions conformes à l'invention peuvent comporter tout support approprié pour l'application topique et compatible avec la substance active, les composés pouvant se trouver dans ce support, soit à l'état dissous, soit à l'état dispersé.

Les compositions destinées à être utilisées en pharmacie se présentent sous forme d'onguents, de teintures, de crèmes, de pommades, de poudres, de timbres, de tampons imbibés, de solutions, d'émulsions, de lotions, de gels, de sprays ou de suspensions. Elles peuvent être, soit anhydres ou aqueuses, selon l'indication clinique.

Les composés sont présents dans ces compositions dans des concentrations comprises entre 0,1 et 10% en poids et en particulier comprises entre 0,2 et 5% en poids.

Les compositions cosmétiques sont notamment destinées à être utilisées sous forme de lotions, de gels, de savons ou de shampooings et contiennent dans un support physiologiquement acceptable, au moins un composé de formule (I) ou l'un de ses sels, esters ou amides.

La concentration des composés de formule (I) est de préférence comprise dans ces cas-là entre 0,01 et 5% en poids, en particulier entre 0,05 et 3% en poids.

Les compositions conformes à l'invention peuvent contenir différents additifs habituellement utilisés en cosmétique ou en pharmacie et qui sont inertes vis-à-vis de la substance active. On peut citer à cet effet des agents hydratants comme la thiamorpholinone et ses dérivés ou l'urée; des agents antiséborrhéiques tels que la S-carboxyméthylcystéine, la S-benzylcystéamine et leurs dérivés, la tioxolone; des agents favorisant la repousse des cheveux comme le phénytoïn (diphényl-5,5 imidazolinedione-2,4) ou encore l'iodure d'oxapropanium, l'acide rétinoïque et ses dérivés, les composés décrits dans les demandes de brevet EP-A-0220118, EP-A-0232199, FR-A-2600064, FR-A-2601002, FR-A-2599031, EP-A-0260162, GB-A-2197316, l'anthraline et ses dérivés; des agents anti-inflammatoires stéroïdiens ou non stéroïdiens; des caroténoïdes et, plus particulièrement, le β-carotène, les acids eicosatétraynoïque-5,8,11,14 et eicosatriynoïque-5,8,11 et leurs esters et amides.

Les compositions peuvent également contenir des agents conservateurs, des agents stabilisants, des agents régulateurs de pH, des agents modificateurs de pression osmotique, des agents émulsifiants, des filtres UV-A et UV-B, des antioxydants tels que l'α-tocophérol, le butylhydroxyanisole ou le butylhydroxytoluène.

L'invention a également pour objet un procédé de traitement cosmétique des cheveux consistant à appliquer sur le cuir chevelu au moins une composition telle que définie ci-dessus.

Un autre objet de l'invention est enfin constitué par l'utilisation des composés de formule (I) dans la préparation d'un médicament pour le traitement de la pelade, de la chute des cheveux, de la dermatite desquamante.

Les exemples suivants sont destinés à illustrer l'invention.

### EXEMPLE 1

### Préparation de la N-(amino-4 chloro-6 oxyde-3 pyrimidinyl-2) N′,N′-diméthylurée et de son isomère N-(amino-2 chloro-6 oxyde-3 pyrimidinyl-4) N′,N′-diméthylurée.

A une suspension de 50 g de diamino-2,4 chloro-6 oxyde-3 pyrimidine dans 500 cm³ de dioxanne anhydre, agitée à température ordinaire, on ajoute rapidement 1,2 équivalent de chlorure de N,N-diméthylcarbamoyle. Le mélange est ensuite porté à une température de 70°C pendant 2 heures. Le produit de départ est totalement transformé. Le mélange est refroidi par un bain de glace. Le produit cristallisé est essoré, lavé à l'éther éthylique, puis séché sous pression réduite.

On obtient 70 g de N-(amino-4 chloro-6 oxyde-3 pyrimidinyl-2) N′,N′-diméthylurée, contenant environ 10% de son isomère, sous forme de cristaux beiges dont le point de fusion est de 192°C.

Le spectre ¹H RMN correspond aux structures attendues.

### EXEMPLE 2

### Préparation de l'amino-7 chloro-5-2H-oxadiazolo-1,2,4 [2,3c] pyrimidinone-2.

A une suspension de 50 g de N-(amino-4 chloro-6 oxyde-3 pyrimidinyl-2) N',N'-diméthylurée dans 500 cm³ de dioxanne agitée à température ordinaire, on ajoute 2 équivalents de chlorure de N,N-diméthylcarbamoyle. Le mélange obtenu est abandonné une nuit à température ambiante, puis porté pendant 24 heures à une température comprise entre 70° et 80°C. A la fin de la réaction, le milieu devient pratiquement homogène. Il est alors versé directement dans 800 cm³ d'éther isopropylique agité à 0°C.

Le produit attendu cristallise. Il est essoré, lavé plusieurs fois à l'éther isopropylique, puis séché sous pression réduite.

On obtient 33,3 g d'amino-7 chloro-5-2H-oxadiazolo-1,2,4 [2,3c] pyrimidinone-2 sous forme de cristaux beiges dont le point de fusion est de 247°C.

Le spectre ¹H RMN correspond à la structure attendue.

| Analyse élémentaire : C₅H₃Cl N₄O₂ | | | | | |
|---|---|---|---|---|---|
| | C | H | N | O | Cl |
| Calculé | 32,19 | 1,62 | 30,03 | 17,15 | 19,01 |
| Trouvé | 32,38 | 1,60 | 30,19 | 16,97 | 18,88 |

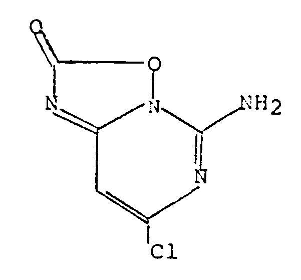

### EXEMPLE 3

### Préparation de la N-(amino-2 chloro-6 oxyde-3 pyrimidinyl-4) N',N'-(pentanediyl)urée.

A une suspension de 300 mg d'amino-7 chloro-5-2H-oxadiazolo-1,2,4 [2,3c] pyrimidinone-2 dans 20 cm³ d'éthanol, agité à température ordinaire sous atmosphère inerte, on ajoute 2 équivalents de pipéridine. La réaction s'effectue en phase hétérogène et au bout de trois heures, le produit de départ est entièrement transformé. Le solide est essoré, lavé à l'éthanol et séché.

On obtient 320 mg de N-(amino-2 chloro-6 oxyde-3 pyrimidinyl-4) N',N'-(pentanediyl)urée sous forme de cristaux jaune pâle, de point de fusion de 226°C.

| Analyse élémentaire : C₁₀H₁₄Cl N₅O₂ | | | | | |
|---|---|---|---|---|---|
| | C | H | Cl | N | O |
| Calculé | 44,20 | 5,19 | 13,05 | 25,73 | 11,78 |
| Trouvé | 44,27 | 5,20 | 13,13 | 25,98 | 12,05 |

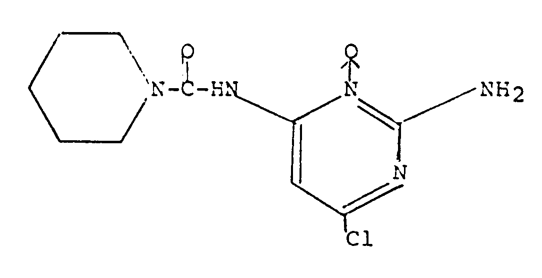

### EXEMPLE 4

### Préparation de la N-(pipéridino-6 amino-2 oxyde-3 pyrimidinyl-4) N′,N′-(pentanediyl)urée.

A une suspension de 200 mg d'amino-7 chloro-5-2H-oxadiazolo-1,2,4 [2,3c] pyrimidinone-2 dans 15 cm³ de n-butanol, on ajoute 2 équivalents de pipéridine. Le mélange agité est porté à une température de 80°C. Un quart d'heure plus tard, le milieu réactionnel est homogène. Après deux heures, le produit de départ est totalement transformé. Le mélange réactionnel est alors versé dans l'eau. La phase organique est décantée, lavée avec une solution d'acide chlorhydrique 1N, puis à l'eau et enfin séchée sur sulfate de sodium.

La solution de butanol est concentrée jusqu'au moment où le produit commence à cristalliser, puis cette solution est glacée. Le solide est essoré et lavé à l'acétone, puis séché.

On obtient 250 mg de N-(pipéridino-6 amino-2 oxyde-3 pyrimidinyl-4) N′,N′-(pentanediyl)urée sous forme de cristaux beiges dont le point de fusion est de 225°C.

Ce produit est analysé sous forme hydratée.

| Analyse élémentaire : C₁₅H₂₄N₆O₂,1/2 H₂O | | | | |
|---|---|---|---|---|
| | C | H | N | O |
| Calculé | 54,86 | 7,37 | 25,59 | 12,18 |
| Trouvé | 54,77 | 7,33 | 25,63 | 11,50 |

Cette urée est également synthétisée en traitant la N-(amino-2 chloro-6 oxyde-3 pyrimidinyl-4) N′,N′-(pentanediyl)urée de l'exemple 3, par 2 équivalents de pipéridine dans le butanol, porté à 80°C. On obtient quantitativement la N-(pipéridino-6 amino-2 oxyde-3 pyrimidinyl-4) N',N'-(pentanediyl)urée.

Cette N-(pipéridino-6 amino-2 oxyde-3 pyrimidinyl-4) N',N'-(pentanediyl)urée est également préparée à partir de l'amino-7 (pipéridino)-5-2H-oxadiazolo-1,2,4 [2,3c] pyrimidinone-2.

On porte une suspension de 1 g de ce produit dans 20 cm³ d'éthanol avec 2 équivalents de pipéridine à 80°C pendant deux heures. Le milieu réactionnel devient très rapidement homogène.

La solution est alors concentrée jusqu'au moment où les premiers cristaux apparaissent, puis on ajoute de l'éther éthylique pour que le reste de produit cristallise. Le solide est essoré, lavé à l'éther et séché.

On obtient 1,25 g de N-(pipéridino-6 amino-2 oxyde-3 pyrimidinyl-4) N',N'-(pentanediyl)urée sous forme d'aiguilles nacrées dont le point de fusion est de 225°C.

| Analyse élémentaire : C₁₅H₂₄N₆O₂ | | | | |
|---|---|---|---|---|
| | C | H | N | O |
| Calculé | 56,43 | 7,55 | 26,23 | 9,99 |
| Trouvé | 56,35 | 7,56 | 26,28 | 10,23 |

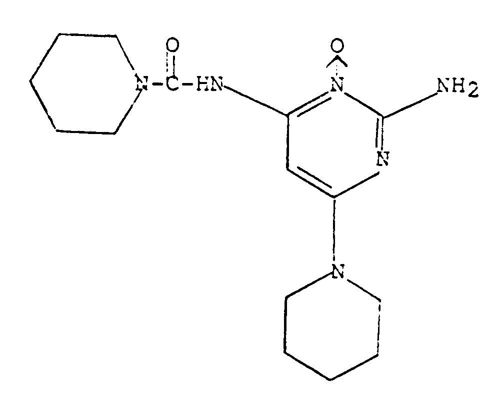

### EXEMPLE 5

### Préparation de la N-(amino-2 chloro-6 oxyde-3 pyrimidinyl-4) N'-(hydroxyéthyl)urée.

A une suspension de 1 g d'amino-7 chloro-5-2H-oxadiazolo-1,2,4 [2,3c] pyrimidinone-2 dans 50 cm³ d'éthanol anhydre, on ajoute 4 équivalents d'éthanol amine. Le mélange agité est porté à une température comprise entre 70° et 80°C pendant six heures. Par refroidissement, le produit attendu cristallise. Il est essoré, lavé à l'éther éthylique, puis séché.

On obtient 0,9 g de N-(amino-2 chloro-6 oxyde-3 pyrimidinyl-4) N'-(hydroxyéthyl)urée sous forme d'une poudre blanche dont le point de fusion est de 254°C.

| Analyse élémentaire : C₇H₁₀Cl N₅O₃ | | | | | |
|---|---|---|---|---|---|
| | C | H | Cl | N | O |
| Calculé | 33,95 | 4,07 | 14,32 | 28,28 | 19,38 |
| Trouvé | 34,20 | 4,11 | 14,51 | 28,20 | 19,40 |

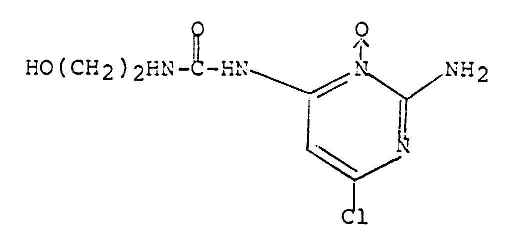

### EXEMPLE 6

### Préparation de la N-(pipéridino-6 amino-2 oxyde-3 pyrimidinyl-4) N'-(hydroxy-2 éthyl)urée.

Une suspension de 0,5 g de N-(amino-2 chloro-6 oxyde-3 pyrimidinyl-4) N'-(hydroxyéthyl)urée dans 20 cm³ de butanol-1 et 0,4 cm³ de pipéridine, est portée à 80°C pendant quatre heures. Temps au bout duquel le produit de départ est entièrement transformé.

Par refroidissement de la solution, le produit attendu cristallise. Il est essoré, lavé à l'éther éthylique et séché.

On obtient 0,350 g de N-(pipéridino-6 amino-2 oxyde-3 pyrimidinyl-4) N'-(hydroxy-2 éthyl)urée sous forme d'une poudre blanche dont le point de fusion est de 248°C.

| Analyse élémentaire : Cl₁₂H₂₀N₆O₃ | | | | |
|---|---|---|---|---|
| | C | H | N | O |
| Calculé | 48,63 | 6,80 | 28,36 | 16,20 |
| Trouvé | 48,61 | 6,81 | 28,39 | 16,23 |

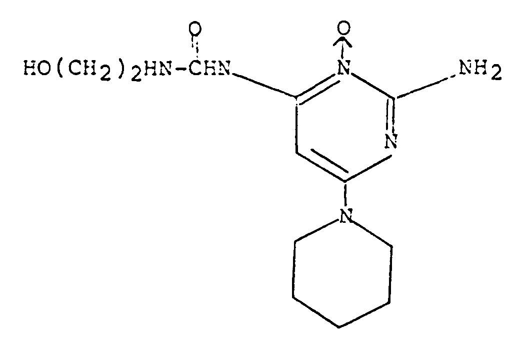

### EXEMPLE 7

### Préparation de la N-(pyrrolidino-6 amino-2 oxyde-3 pyrimidinyl-4) N',N'-(butanediyl)urée.

A une suspension de 2 g d'amino-7 chloro-5-2H-oxadiazolo-1,2,4 [2,3c] pyrimidinone-2 dans 50 cm³ d'éthanol, on ajoute 4 équivalents de pyrrolidine. Ce mélange agité est porté à 80°C pendant 2 heures. Le milieu devient rapidement homogène.

Par refroidissement, le produit attendu cristallise, il est essoré, lavé à l'éther éthylique et séché.

On obtient 1,9 g de N-(pyrrolidino-6 amino-2 oxyde-3 pyrimidinyl-4) N',N'-(butanediyl)urée sous forme de paillettes nacrées blanches dont le point de fusion est de 240°C.

| Analyse élémentaire : C₁₃H₂₀N₆O₂ | | | | |
|---|---|---|---|---|
| | C | H | N | O |
| Calculé | 53,41 | 6,90 | 28,75 | 10,94 |
| Trouvé | 53,31 | 6,96 | 28,23 | 11,22 |

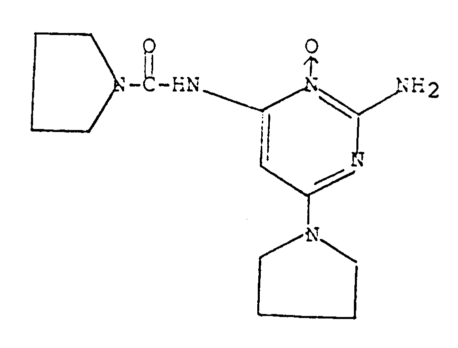

### EXEMPLE 8

### Préparation de la N-(amino-2 chloro-6 oxyde-3 pyrimidinyl-4) N'-(dihydroxy-2,3 propyl)urée.

A une suspension de 2 g d'amino-7 chloro-5-2H-oxadiazolo-1,2,4 [2,3] pyrimidinone-2 dans 50 cm³ d'éthanol, on ajoute 1,1 équivalent de dihydroxy-2,3 propylamine. Sous agitation, ce mélange est porté trois heures sous reflux. A ce stade, le produit de départ est transformé.

Par refroidissement, le produit attendu cristallise. Il est essoré, lavé à l'éther éthylique et séché.

On obtient 1,4 g de N-(amino-2 chloro-6 oxyde-3 pyrimidinyl-4) N'-(dihydroxy-2,3 propyl)urée sous forme de cristaux blancs dont le point de fusion est de 219°C.

| Analyse élémentaire : C₈H₁₂Cl N₅O₄ | | | | | |
|---|---|---|---|---|---|
| | C | H | Cl | N | O |
| Calculé | 34,61 | 4,36 | 12,77 | 25,22 | 23,05 |
| Trouvé | 34,73 | 4,08 | 12,69 | 25,17 | 23,23 |

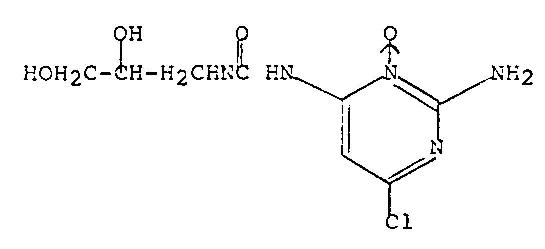

### EXEMPLE 9

### Préparation du N-(pipéridino-6 amino-2 oxyde-3 pyrimidinyl-4) N'-(dihydroxy-2,3 propyl)urée.

Un mélange de 1 g de N-(amino-2 chloro-6 oxyde-3 pyrimidinyl-4) N'-(dihydroxy-2,3 propyl)urée et de 0,7 cm³ de pipéridine dans 50 cm³ d'éthanol, est porté sous reflux pendant 30 heures, temps au bout duquel la majorité du produit de départ est transformée.

Par refroidissement, le produit attendu cristallise. Il est essoré, lavé à l'éther éthylique et séché.

On obtient 1 g de N-(pipéridino-6 amino-2 oxyde-3 pyrimidinyl-4) N'-(dihydroxy-2,3 propyl)urée sous forme de cristaux blancs de point de fusion 224°C qui sont analysés sous forme hydratée.

| Analyse élémentaire : C₁₃H₂₂N₆O₄, 1/2 H₂O | | | | |
|---|---|---|---|---|
| | C | H | N | O |
| Calculé | 46,70 | 6,63 | 25,14 | 21,53 |
| Trouvé | 46,72 | 6,54 | 24,89 | 21,66 |

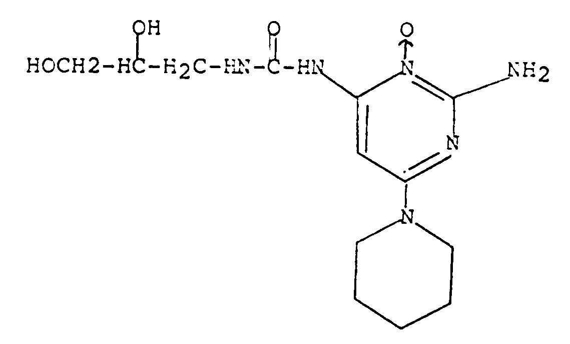

### EXEMPLE 10

### Préparation du N-(morpholino-6 amino-2 oxyde-3 pyrimidinyl-4) N′,N′-(oxa-3 pentanediyle)urée.

Un mélange de 2 g d'amino-7 chloro-5-2H-oxadiazolo-1,2,4 [2,3c] pyrimidinone-2 et de 3,7 cm³ de morpholine dans 50 cm³ d'éthanol, est porté à une température d'environ 70°C pendant deux heures.

Par refroidissement du mélange à 0°C, le produit attendu cristallise. Il est essoré, lavé plusieurs fois à l'éther éthylique et séché.

On obtient 2,6 g de N-(morpholino-6 amino-2 oxyde-3 pyrimidinyl-4) N′,N′-(oxa-3 pentanediyle)urée partiellement sous forme de chlorhydrate.

Ce produit est agité dans 100 cm³ d'eau. Par addition de bicarbonate de sodium, on amène le pH de la solution vers 8. Le produit attendu est alors extrait trois fois au chlorure de méthylène. Ces phases organiques sont rassemblées, lavées à l'eau et séchées sur sulfate de sodium, puis concentrées.

Le solide est recristallisé dans l'éthanol.

On obtient 1,5 g de N-(morpholino-6 amino-2 oxyde-3 pyrimidinyl-4) N′,N′-(oxa-3 pentanediyle)urée sous forme de paillettes nacrées blanches dont le point de fusion est supérieur à 260°C.

Ce produit est analysé sous forme hydratée.

| Analyse élémentaire : C₁₃H₂₀N₆O₄, 1/4 H₂O | | | | |
|---|---|---|---|---|
| | C | H | N | O |
| Calculé | 47,48 | 6,28 | 25,56 | 20,68 |
| Trouvé | 47,41 | 6,28 | 25,47 | 21,30 |

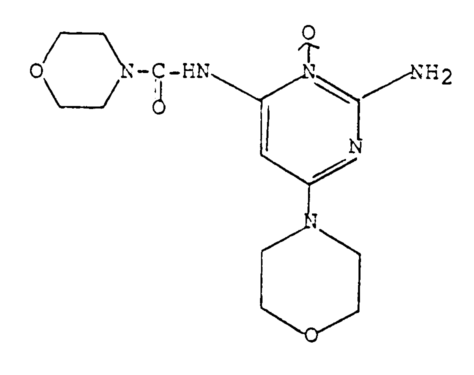

### EXEMPLE 11

### Préparation de la N-(pipéridino-6 amino-4 oxyde-3 pyrimidinyl-2) N'-(hydroxyéthylurée).

A une suspension de 0,5 g d'amino-7 pipéridino-5-2H-oxadiazolo-1,2,4 [2,3a] pyrimidinone-2 dans 20 cm³ de butanol-1, on ajoute 0,5 cm³ d'éthanolamine. Le mélange ainsi obtenu est placé sous agitation à une température d'environ 90°C.

Le milieu devient progressivement homogène. Au bout de quatre heures, le produit de départ est transformé. Le butanol est éliminé par évaporation sous vide et le produit obtenu est agité dans l'acétone. Le solide est essoré, lavé à l'acétone puis séché.

On obtient 0,5 g de N-(pipéridino-6 amino-4 oxyde-3 pyrimidinyl-2) N'-(hydroxyéthylurée) sous forme de cristaux blancs dont le point de fusion est de 174°C.

| Analyse élémentaire : C₁₂H₂₀N₆O₃, 1/4 H₂O | | | | |
|---|---|---|---|---|
| | C | H | N | O |
| Calculé | 47,91 | 6,87 | 27,94 | 17,28 |
| Trouvé | 47,66 | 6,41 | 27,43 | 17,55 |

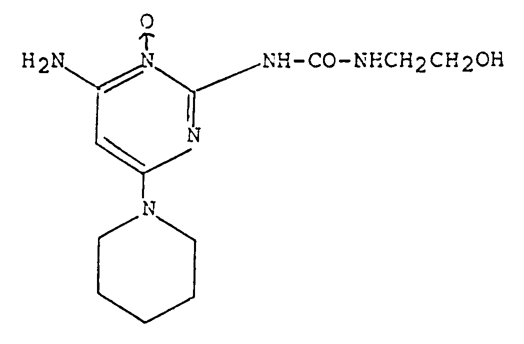

### EXEMPLE 12

### Préparation du N-(pipéridino-6 amino-2 oxyde-3 pyrimidinyl-4) N'-(méthylglucamine)urée.

A une suspension de 11 g d'amino-7 (pipéridino)-5-2H-oxadiazolo-1,2,4 [2,3c] pyrimidinone-2 dans 300 cm³ d'éthanol, agitée à température ambiante, on ajoute 9,1 g de N-méthylglucamine. Le mélange est alors porté à 80°C pendant quatre heures. Le milieu réactionnel devient progressivement homogène. Le produit de départ étant totalement transformé, la solution est refroidie à 0°C. Le produit attendu cristallise. Il est essoré et séché.

On obtient 14,5 g de N-(pipéridino-6 amino-2 oxyde-3 pyrimidinyl-4) N'-(méthylglucamine)urée sous forme de cristaux blancs dont le point de fusion est de 186°C.

| Analyse élémentaire : C₁₇H₃₀N₆O₇ | | | | |
|---|---|---|---|---|
| | C | H | N | O |
| Calculé | 47,43 | 7,02 | 19,52 | 26,02 |
| Trouvé | 46,90 | 7,06 | 19,27 | 27,05 |

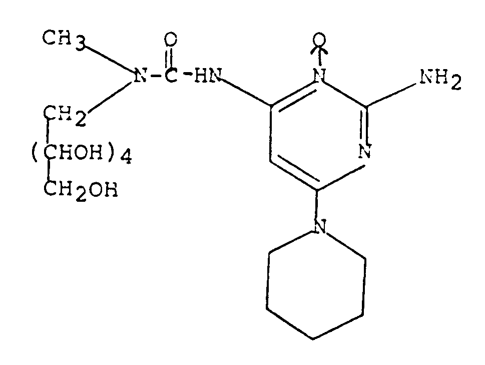

### EXEMPLE 13

### Préparation de la N-(hydroxy-5'oxa-3'pentylamino-6 amino-2 oxyde-3 pyrimidinyl-4)N'-(hydroxy-5 oxa-3 pentyl)urée.

A une suspension de 2 g d'amino-7 chloro-5 en 2H-oxadiazolo-1,2,4 [2,3c] pyrimidinone-2 dans 50 cm³ de butanol, on ajoute 2,5 équivalents de 2-(2-aminoéthoxy)-éthanol ou "diglycolamine".

Ce mélange est alors agité 4 heures à température ordinaire puis placé ensuite 4 heures sous reflux. Le solvant est alors éliminé par évaporation sous vide. Le produit brut est alors dispersé dans 20 cm³ d'éthanol par agitation à température ordinaire. Le solide est essoré puis séché. On obtient 1,5 g de N-(hydroxy-5'oxa-3'pentylamino-6 amino-2 oxyde-3 pyrimidinyl-4)N'-(hydroxy-5 oxa-3 pentyl)urée sous forme d'une poudre blanche dont le point de fusion est de 214°C.

| Analyse élémentaire : C₁₃H₂N₆O₆ | | | | |
|---|---|---|---|---|
| | C | H | N | O |
| Calculé | 43,33 | 6,71 | 23,32 | 26,64 |
| Trouvé | 43,55 | 6,69 | 23,38 | 26,48 |

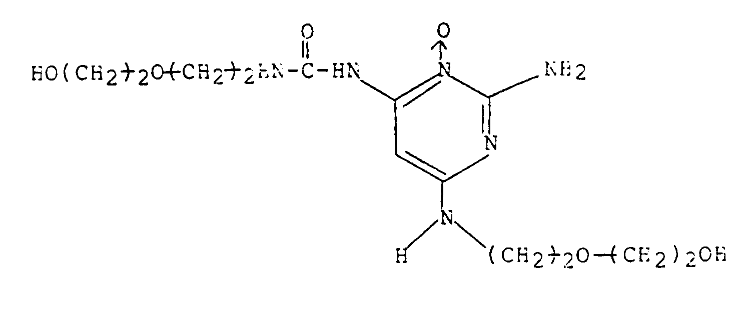

### EXEMPLES DE COMPOSITIONS

1) On prépare une lotion de composition suivante :

| | |
|---|---|
| - N-(pipéridino-6 amino-2 oxyde-3 pyrimidinyl-4) N′-(hydroxy-2 éthyl)urée | 2 g |
| - Propylèneglycol | 20 g |
| - Alcool éthylique | 50 g |
| - Eau | qsp 100 g |

2) On prépare la lotion destinée à stimuler la repousse des cheveux de la composition suivante :

| | |
|---|---|
| - N-(pipéridino-6 amino-2 oxyde-3 pyrimidinyl-4) N′-(méthylglucamine)urée | 5 g |
| - Propylèneglycol | 20 g |
| - Ethanol | 50 g |
| - Eau | qsp 100 g |

1 à 2 ml de ces lotions sont appliqués sur les zones alopéciques du cuir chevelu; ces applications, éventuellement accompagnées par un massage pour favoriser la pénétration, étant effectuées une ou deux fois par jour.
3) On prépare la composition suivante sous forme de gel :

| | |
|---|---|
| - N-(pipéridino-6 amino-2 oxyde-3 pyrimidinyl-4) N′-(méthylglucamine)urée | 2 g |
| - Hydroxypropylcellulose vendue par la Société HERCULES sous la dénomination commerciale "KLUCEL G" | 3 g |
| - Propylèneglycol | 20 g |
| - Ethanol | 50 g |
| - Conservateur qs | |
| - Eau | qsp 100 g |

4) On prépare la composition suivante sous forme de gel :

| | |
|---|---|
| - N-(pipéridino-6 amino-2 oxyde-3 pyrimidinyl-4) N′-(dihydroxy-2,3 propyl)urée | 3 g |
| - Méthylhydroxypropylcellulose vendue par la Société DOW CHEMICAL sous la dénomination commerciale "METHOCEL F" | 1 g |
| - Ethanol | 40 g |
| - Eau | qsp 100 g |

## Revendications (Revendications pour l'(les) Etat(s) contractant(s) suivant(s): AT, BE, CH, DE, FR, GB, IT, LI, NL, SE)

1. Composé caractérisé par le fait qu'il répond à la formule : 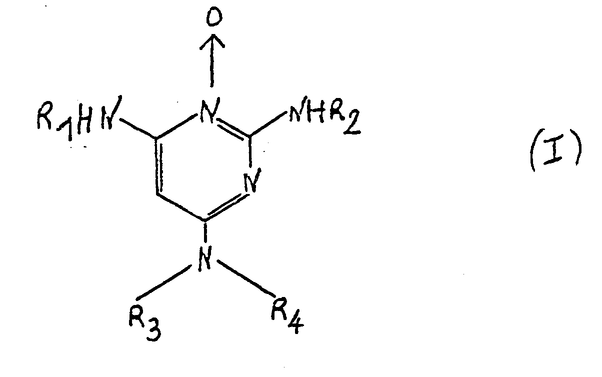 dans laquelle :
R₃ et R₄, identiques ou différents, désignent un atome d'hydrogène, un radical alkyle, linéaire ou ramifié en C₁-C₁₈, alcényle ayant 2 à 18 atomes de carbone, cycloalkyle ayant de 5 à 8 atomes de carbone; éventuellement substitués par un ou plusieurs groupements alkyle inférieur;
les groupements alkyle, alcényle ou cycloalkyle pouvant eux-mêmes être substitués par un ou plusieurs groupement(s) hydroxyle,
R₃ et/ou R₄ désignent également un groupement aryle ou aralkyle, répondant à la formule : 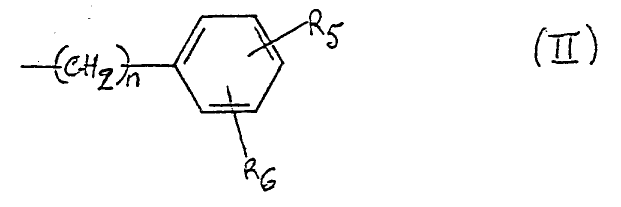 dans laquelle :
n peut prendre les valeurs de 0 à 4 et où R₅ et/ou R₆, indépendamment l'un de l'autres désignent hydrogène, un groupement alkyle en C₁-C₆, un groupement nitro, hydroxyle ou un atome d'halogène, ou encore un groupement carboxylique, ainsi que les formes salifiées, esters et amides de ces derniers,
R₃ et R₄, pris ensemble avec l'atome d'azote auquel ils sont reliés, peuvent former un hétérocycle de 3 à 7 atomes de carbone,
R₁ et R₂, désignent un atome d'hydrogène ou un groupement carbamoyle, de formule : 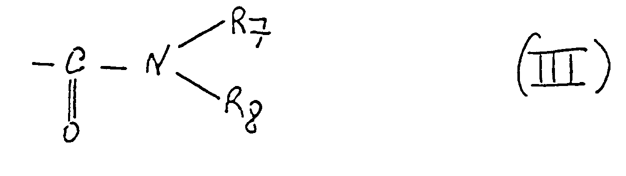 sous réserve que l'un désigne un hydrogène et l'autre un groupement carbamoyle ;
R₇ et/ou R₈ désignent, indépendamment l'un de l'autre, un atome d'hydrogène, un radical alkyle, linéaire ou ramifié en C₁-C₁₈, substitué par un ou plusieurs groupement(s) hydroxyle et/ou amino, cette chaîne alkyle pouvant être éventuellement interrompue par un ou plusieurs hétéroatome(s) choisi(s) parmi le soufre, l'azote ou l'oxygène,
R₇ et R₈ désignent également un groupe cycloalkyle substitué par un groupement hydroxyle, amino et/ou carboxylique,
R₇ et R₈ peuvent former avec l'atome d'azote auquel ils sont rattachés, un hétérocycle choisi parmi les groupements morpholino, pipéridino, pyrrolidino, pipérazino, N-alkyl 4'-pipérazino, dans lequel le groupement alkyle en position 4' contient de 1 à 6 atomes de carbone, dont l'un peut être substitué par un groupement hydroxyle ou thiazolidine, ainsi que leur forme tautomère répondant aux formules :

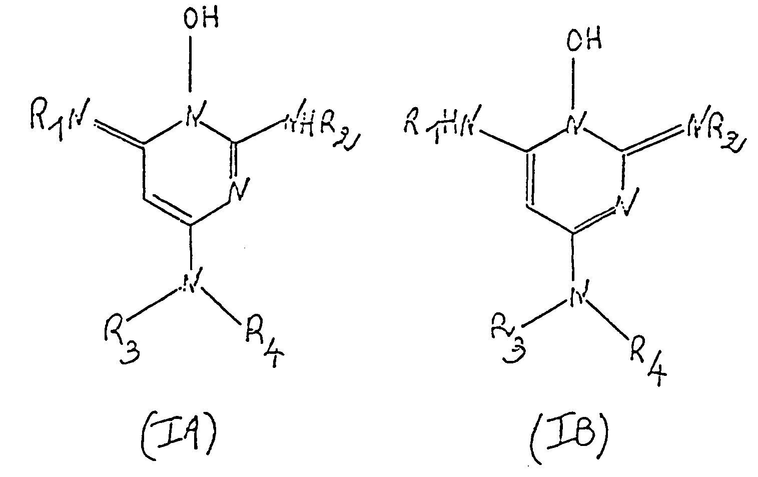

2. Composé selon la revendication 1, caractérisé par le fait que dans la formule (I), le radical alkyle en C₁-C₁₈ substitué par un ou plusieurs groupement(s) hydroxyle ou amino, représenté par le le radical R₇ ou R₈, est choisi parmi les radicaux hydroxy-2 éthyle, dihydroxy-2,3 propyle, hydroxy-2 propyle, hydroxy-2 éthoxyéthyle, hydroxy-2 éthylaminoéthyle, amino-2 éthyle.

3. Composé selon l'une quelconque des revendications 1 à 2, caractérisé par le fait qu'il répond à la formule (la) suivante : 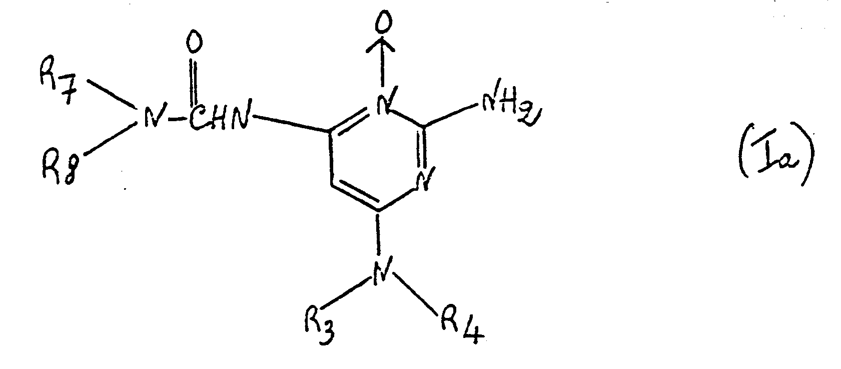 dans laquelle R₃, R₄, R₇ et R₈ ont la signification indiquée dans la revendication 1.

4. Composé selon la revendication 1 ou 2, caractérisé par le fait qu'il répond aux formules (Ib) et (Ic) : 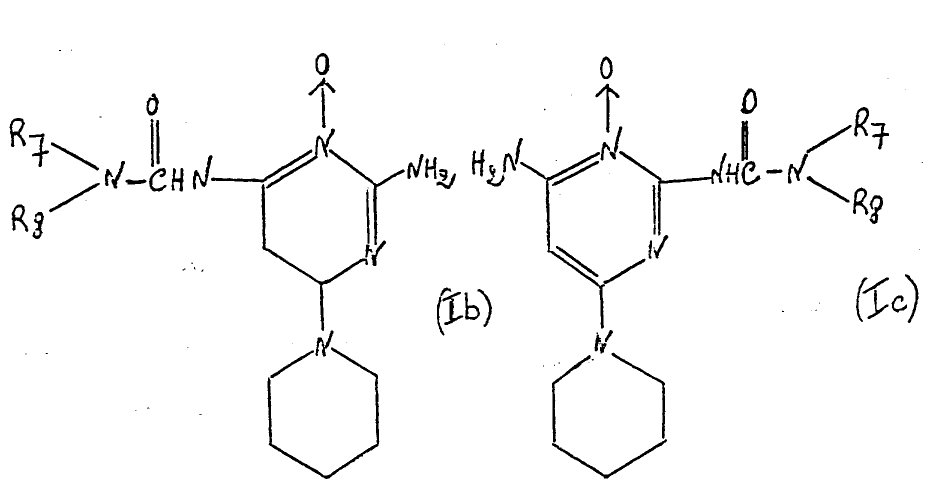 dans lesquelles R₇ et R₈ ont les significations indiquées dans la revendication 1.

5. Composé intermédiaire servant à la préparation des composés selon l'une quelconque des revendications 1 à 4, caractérisé par le fait qu'il répond aux formules :

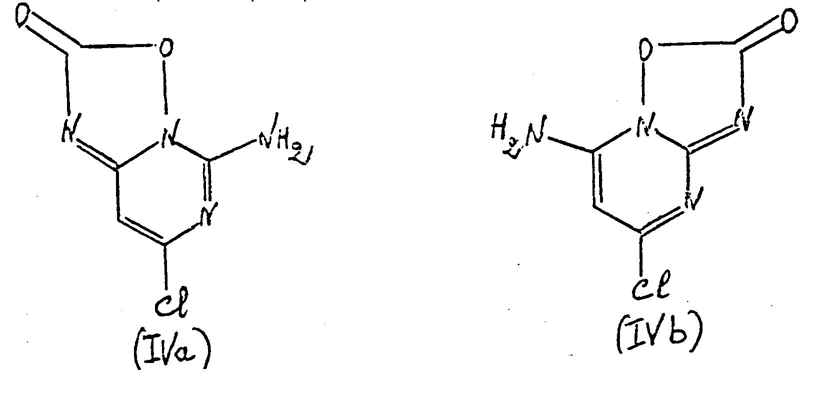

6. Procédé de préparation des composés selon l'une quelconque des revendications 1 à 4, caractérisé par le fait que l'on traite le composé selon la revendication 5 par une amine de formule : 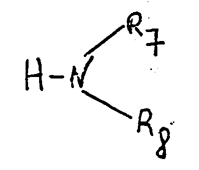 dans laquelle R₇ et R₈ ont les significations indiquées dans la revendication 1,
que l'on traite le dérivé monourée résultant par une amine de formule : 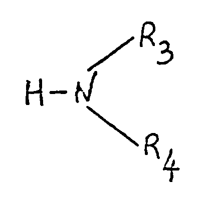 dans laquelle R₃ et R₄ ont les significations définies dans la revendication 1.

7. Procédé de préparation des composés selon la revendication 4, répondant à la formule (Ib) : 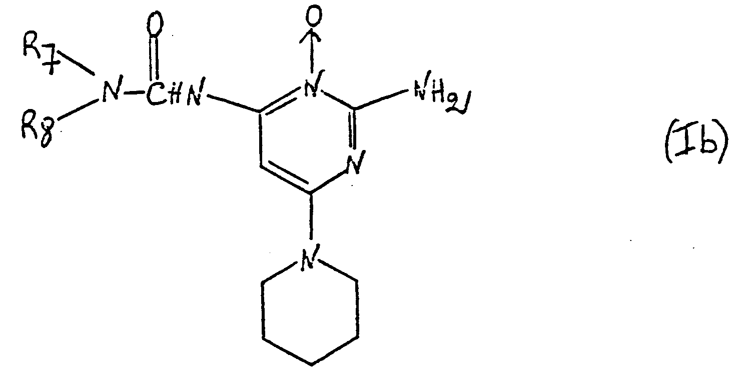 caractérisé par le fait que l'on traite l'amino-7 pipéridino-5-2H-oxadiazolo-1,2,4 [2,3c] pyrimidinone-2 de formule (VIa) : 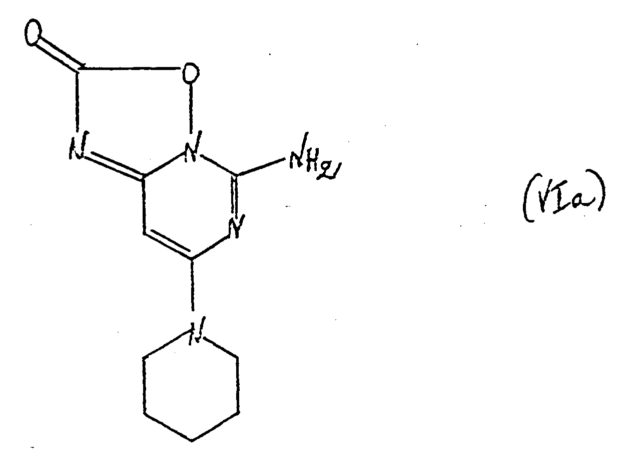 par l'amine de formule : 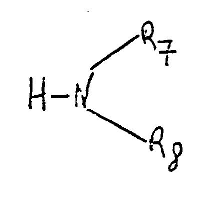 dans laquelle R₇ et R₈ ont les significations définies dans la revendication 1.

8. Procédé de préparation du composé selon la revendication 4, répondant à la formule (Ic) : 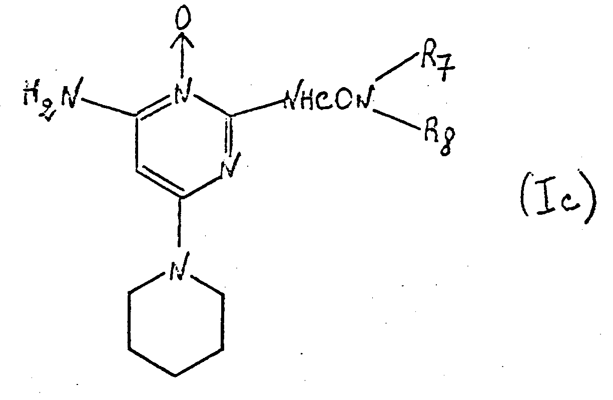 dans laquelle R₇ ou R₈ désigne un hydrogène, caractérisé par le fait que l'on traite l'amino-7 pipéridino-5-2H-oxadiazolo-1,2,4 [2,3a] pyrimidinone-2 de formule (VIb) : 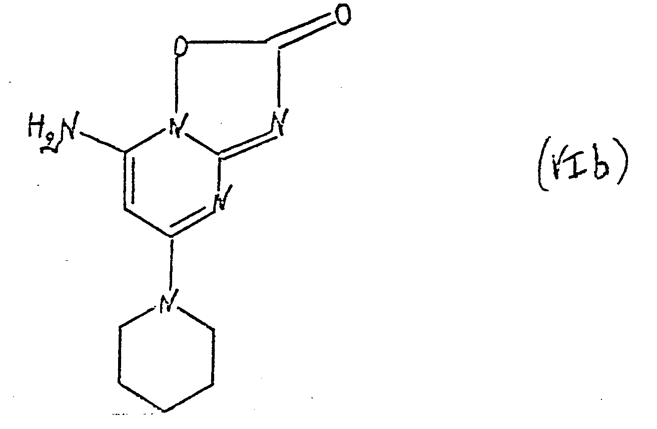 par une amine primaire de formule : 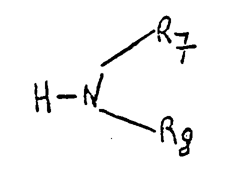 dans laquelle R₇ et R₈ ont les significations indiquées ci-dessus.

9. Procédé de préparation des composés intermédiaires de formules (IVa) et (IVb) selon la revendication 5, caractérisé par le fait que l'on traite dans une première étape, la diamino-2,4 chloro-6 oxyde-3 pyrimidine de formule (VII) : 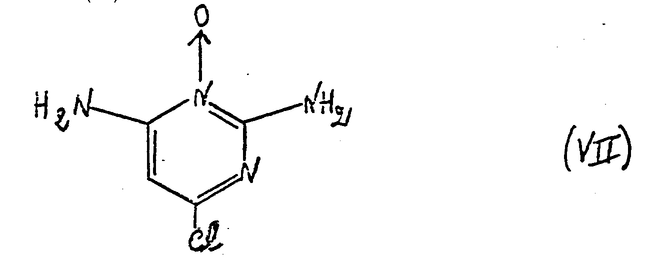 par un chlorure de carbamoyle de formule (VIII) : 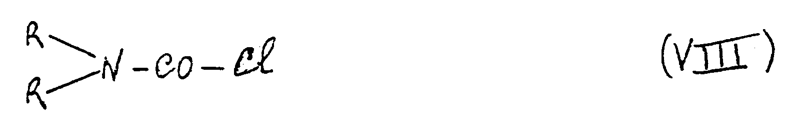 dans laquelle R désigne un groupement alkyle, dans un solvant polaire aprotique, choisi parmi le diméthylsulfoxyde, le tétrahydrofuranne ou le dioxanne, pour obtenir le mélange de dérivés monourées isomères de formules (IXa) et (IXb) : 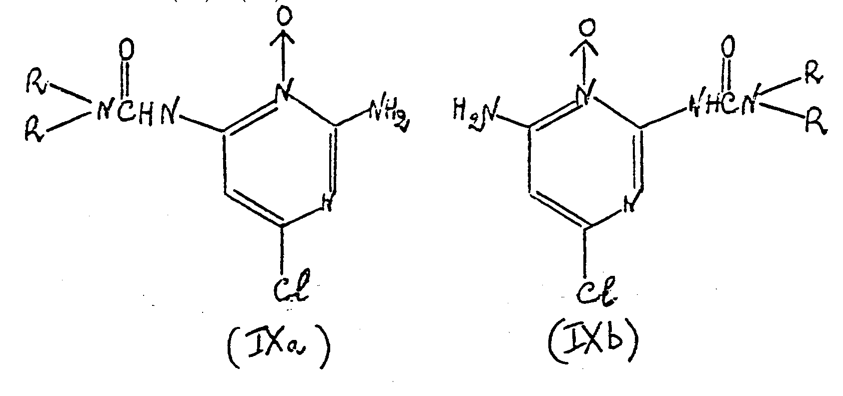 que l'on traite cedit mélange par un excès de chlorure de carbamoyle (formule VIII) pour obtenir l'amino-7 chloro-5-2H-oxadiazolo-1,2,4 [2,3c] pyrimidinone-2 de formule (IVa) et son isomère de formule (IVb).

10. Composition cosmétique ou pharmaceutique, caractérisée par le fait qu'elle contient dans un support approprié pour une application topique, au moins un composé répondant à l'une quelconque des revendications 1 à 4.

11. Composition pharmaceutique selon la revendication 10, caractérisée par le fait qu'elle se présente sous forme d'onguents, de teintures, de crèmes, de pommades, de poudres, de timbres, de tampons imbibés, de solutions, d'émulsions, de lotions, de gels, de sprays ou de suspensions anhydres ou aqueuses, contenant au moins un composé tel que défini dans l'une quelconque des revendications 1 à 4.

12. Composition selon la revendication 11, caractérisée par le fait que les composés de formule (I) sont présents dans des concentrations comprises entre 0,1 et 10% en poids par rapport au poids total de la composition.

13. Composition cosmétique selon la revendication 10, sous forme de lotions, de gels, de savons, de shampooings, caractérisée par le fait qu'elle contient dans un support physiologiquement acceptable, au moins l'un des composés tels que définis dans l'une quelconque des revendications 1 à 4, à une concentration comprise entre 0,01 et 5% en poids.

14. Composition selon la revendication 13, caractérisée par le fait qu'elle contient en plus des agents hydratants, des agents antiséborrhéiques, des agents favorisant la repousse des cheveux, des agents anti-inflammatoires, stéroïdiens ou non stéroïdiens, des caroténoïdes, des acides eicosatétraynoïque-5,8,11,14 et eicosatriynoïque-5,8,11 et leurs esters et amides.

15. Composition selon l'une quelconque des revendications 10 à 14, caractérisée par le fait qu'elle contient également des agents conservateurs, des agents stabilisants, des agents régulateurs de pH, des agents modificateurs de pression osmotique, des agents émulsifiants, des filtres UV-A et UV-B, des agents antioxydants.

16. Procédé de traitement cosmétique des cheveux, caractérisé par le fait qu'on applique sur le cuir chevelu au moins une composition telle que définie dans l'une quelconque des revendications 10 à 15.

17. Utilisation des composés selon l'une quelconque des revendications 1 à 4, pour la préparation d'un médicament pour le traitement de la pelade, de la chute des cheveux, de la dermatite desquamante.

## Revendications (Revendications pour l'(les) Etat(s) contractant(s) suivant(s): ES)

1. Procédé de préparation du composé qui répond à la formule : 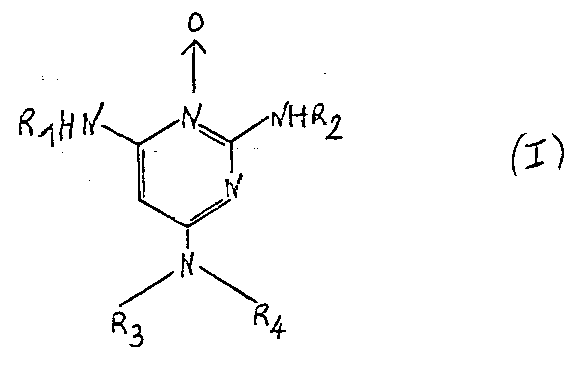 dans laquelle :
R₃ et R₄, identiques ou différents, désignent un atome d'hydrogène, un radical alkyle, linéaire ou ramifié en C₁-C₁₈, alcényle ayant 2 à 18 atomes de carbone, cycloalkyle ayant de 5 à 8 atomes de carbone; éventuellement substitués par un ou plusieurs groupements alkyle inférieur;
les groupements alkyle, alcényle ou cycloalkyle pouvant eux-mêmes être substitués par un ou plusieurs groupement(s) hydroxyle,
R₃ et/ou R₄ désignent également un groupement aryle ou aralkyle, répondant à la formule : 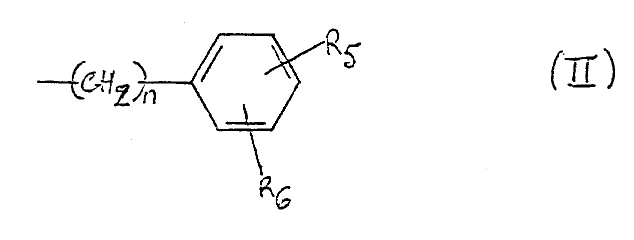 dans laquelle :
n peut prendre les valeurs de 0 à 4 et où R₅ et/ou R₆, indépendamment l'un de l'autre, désignent hydrogène, un groupement alkyle en C₁-C₆, un groupement nitro, hydroxyle ou un atome d'halogène, ou encore un groupement carboxylique, ainsi que les formes salifiées, esters et amides de ces derniers,
R₃ et R₄, pris ensemble avec l'atome d'azote auquel ils sont reliés, peuvent former un hétérocycle de 3 à 7 atomes de carbone,
R₁ et R₂, désignent un atome d'hydrogène ou un groupement carbamoyle, de formule : 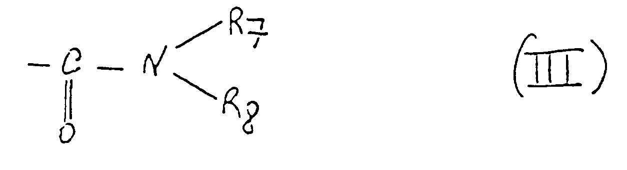 sous réserve que l'un désigne un hydrogène et l'autre un groupement carbamoyle ;
R₇ et/ou R₈ désignent, indépendamment l'un de l'autre, un atome d'hydrogène, un radical alkyle, linéaire ou ramifié en C₁-C₁₈, substitué par un ou plusieurs groupement(s) hydroxyle et/ou amino, cette chaîne alkyle pouvant être éventuellement interrompue par un ou plusieurs hétéroatome(s) choisi(s) parmi le soufre, l'azote ou l'oxygène,
R₇ et R₈ désignent également un groupe cycloalkyle substitué par un groupement hydroxyle, amino et/ou carboxylique,
R₇ et R₈ peuvent former avec l'atome d'azote auquel ils sont rattachés, un hétérocycle choisi parmi les groupements morpholino, pipéridino, pyrrolidino, pipérazino, N-alkyl 4'-pipérazino, dans lequel le groupement alkyle en position 4' contient de 1 à 6 atomes de carbone, dont l'un peut être substitué par un groupement hydroxyle ou thiazolidine, ainsi que leur forme tautomère répondant aux formules : 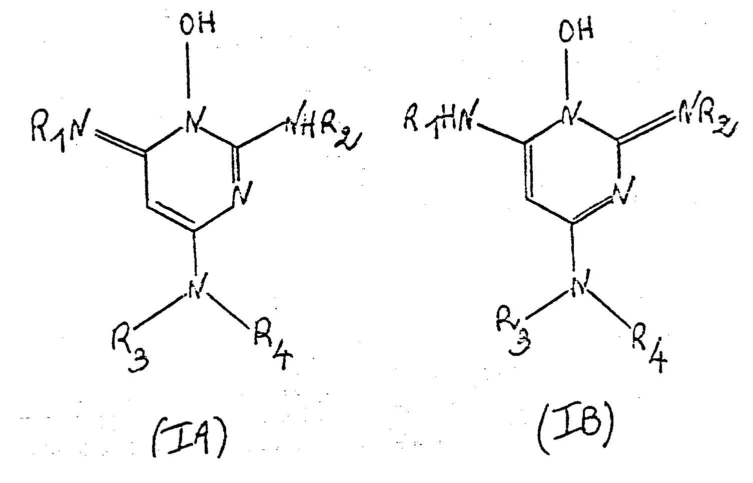 caractérisé par le fait que :
(A) pour préparer des composés répondant à la formule (IA) : 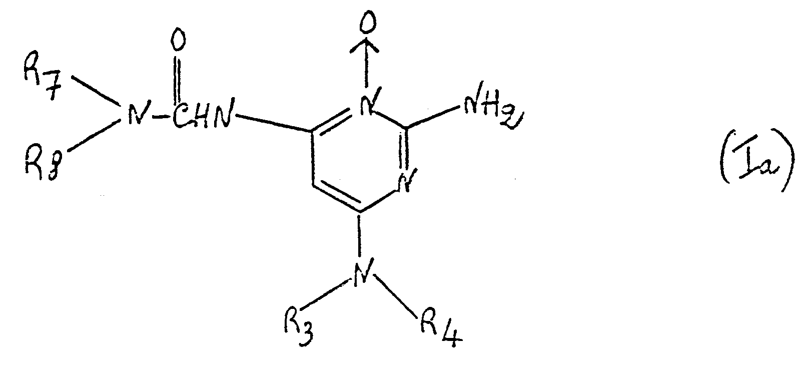 dans laquelle R₃,R₄,R₇ et R₈ ont la signification indiquée ci-dessus ou leurs isomères, on traite l'amino-7 chloro-5 oxadiazolo-1,2,4 [2,3c] pyrimidinone-2 de formule (IVa) ou son isomère de formule (IVb) : 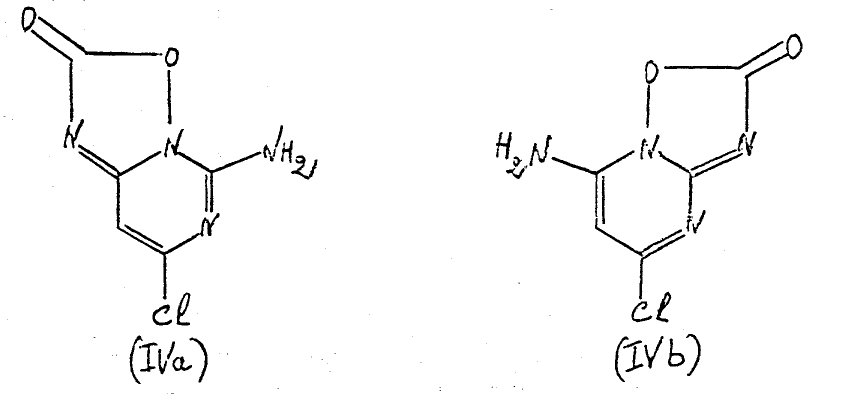 par une amine de formule : 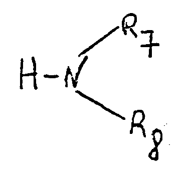 dans laquelle R₇ et R₈ ont les significations indiquées ci-dessus, dans une première étape puis on traite le dérivé monourée résultant par une amine de formule : 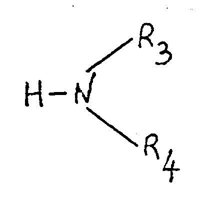 dans laquelle R₃ et R₄ ont les significations indiquées ci-dessus ;
(B) pour préparer les composés répondant à la formule (Ib) : 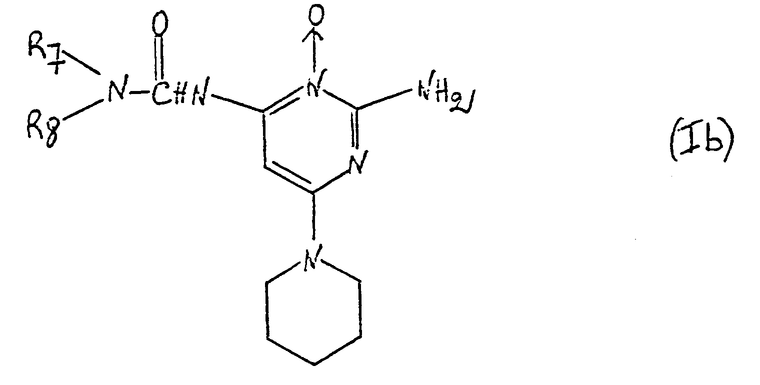 dans laquelle R₇ et R₈ ont les significations indiquées ci-dessus, on traite l'amino-7 pipéridino-5-2H-oxadiazolo-1,2,4 [2,3c] pyrimidinone-2 de formule (VIa) : 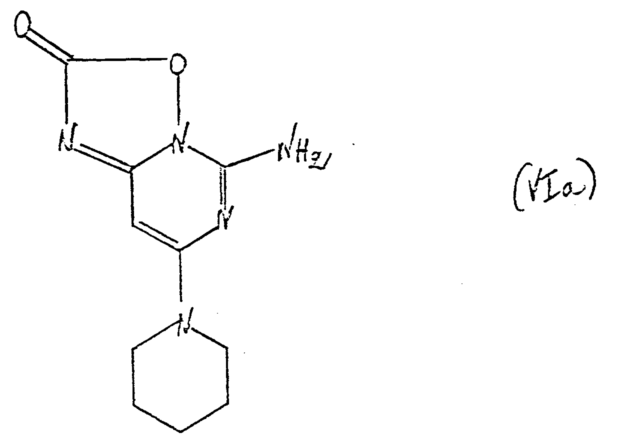 par l'amine de formule : 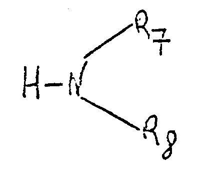
(C) pour préparer les composés répondant à la formule (Ic) : 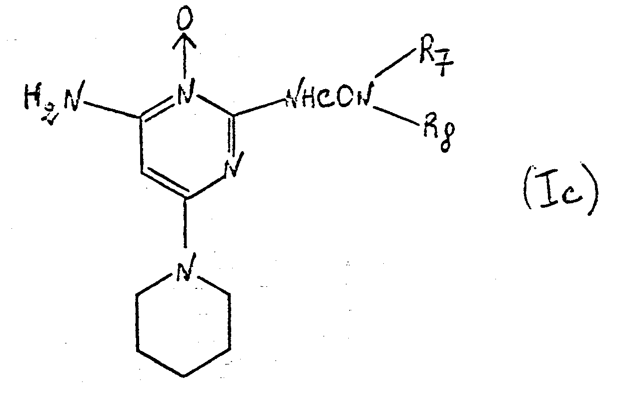 dans laquelle R₇ et R₈ ont les significations indiquées ci-dessus, on traite l'amino-7 pipéridino-5-2H-oxadiazolo-1,2,4 [2,3c] pyrimidinone-2 de formule (VIb) : 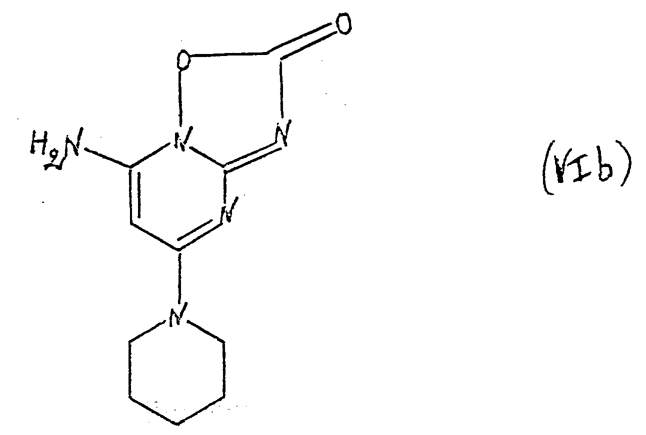 par une amine primaire de formule :

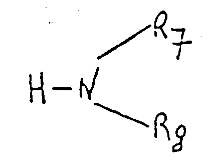

2. Procédé selon la revendication 1, caractérisé par le fait que dans la formule (I), le radical alkyle en C₁-C₁₈ substitué par un ou plusieurs groupement(s) hydroxyle ou amino, représenté par le radical R₇ ou R₈, est choisi parmi les radicaux hydroxy-2 éthyle, dihydroxy-2,3 propyle, hydroxy-2 propyle, hydroxy-2 éthoxyéthyle, hydroxy-2 éthylaminoéthyle, amino-2 éthyle.

3. Procédé de préparation des composés intermédiaires de formules (IVa) et (IVb) tels que définis dans la revendication 1, caractérisé par le fait que l'on traite dans une première étape, la diamino-2,4 chloro-6 oxyde-3 pyrimidine de formule (VII) : 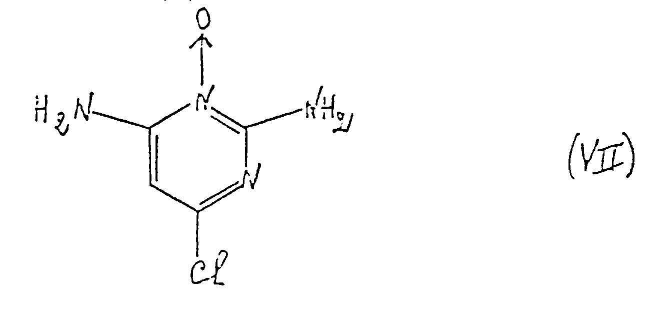 par un chlorure de carbamoyle de formule (VIII) : 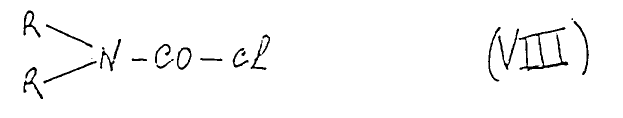 dans laquelle R désigne un groupement alkyle, dans un solvant polaire aprotique, choisi parmi le diméthyl-sulfoxyde, le tétrahydrofuranne ou le dioxanne, pour obtenir le mélange de dérivés monourées isomères de formules (IXa) et (IXb) : 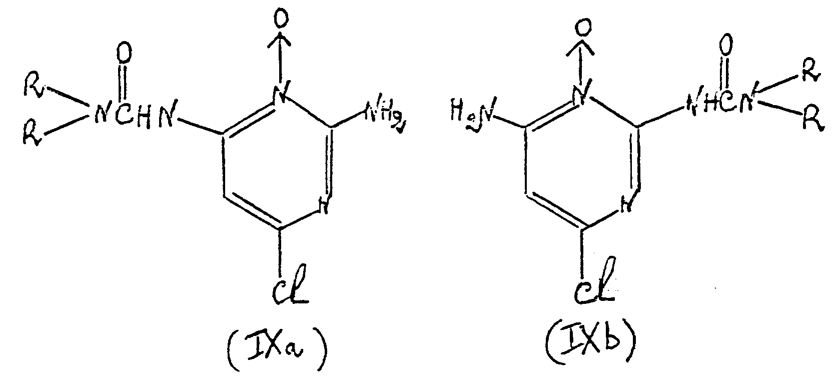 que l'on traite cedit mélange par un excès de chlorure de carbamoyle (formule VIII) pour obtenir l'amino-7 chloro-5-2H-oxadiazolo-1,2,4 [2,3c] pyrimidinone-2 de formule (IVa) et son isomère de formule (IVb).

4. Composition cosmétique sous forme de lotion, de gel, de savon, de shampooing, caractérisée par le fait qu'elle contient dans un support physiologiquement acceptable approprié pour une application topique, au moins un des composés tels que définis dans l'une quelconque des revendications 1 à 2, dans une concentration comprise entre 0,01 et 5% en poids par rapport au poids total de la composition.

5. Composition selon la revendication 4, caractérisée par le fait qu'elle contient en plus des agents hydratants, des agents antiséborrhéiques, des agents favorisant la repousse des cheveux, des agents anti-inflammatoires, stéroïdiens ou non stéroïdiens, des caroténoïdes, des acides eicosatétraynoïque-5,8,11,14 et eicosatriynoïque-5,8,11 et leurs esters et amides.

6. Composition selon l'une quelconque des revendications 4 à 5, caractérisée par le fait qu'elle contient également des agents conservateurs, des agents stabilisants, des agents régulateurs de pH, des agents modificateurs de pression osmotique, des agents émulsifiants, des filtres UV-A et UV-B, des agents antioxydants.

7. Procédé de préparation d'une composition pharmaceutique sous forme d'onguent, de teinture, de crème, de pommade, de poudre, de timbre, de tampon imbibé, de solution, d'émulsion, de gel, de spray, de suspension anhydre ou aqueuse, destinée au traitement des maladies du cuir chevelu, caractérisé par le fait que l'on incorpore dans un support pharmaceutiquement acceptable, approprié pour une application topique, au moins un composé selon la revendication 1 ou 2 dans des concentrations comprises entre 0,1 et 10% en poids.

## Claims (Claims for the following Contracting State(s): AT, BE, CH, DE, FR, GB, IT, LI, NL, SE)

1. A compound characterised in that it has the formula: 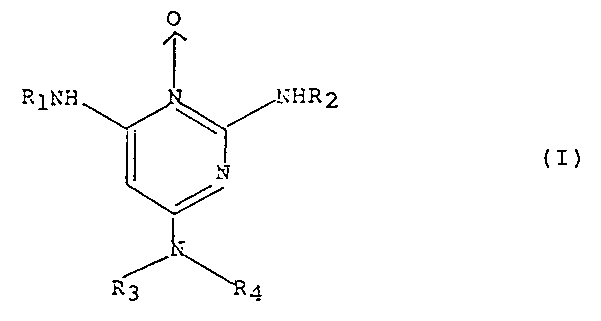 wherein:
R₃ and R₄ may be identical or different and represent a hydrogen atom, a C₁-C₁₈ linear or branched alkyl radical, an alkenyl radical having two to 18 carbon atoms, a cycloalkyl radical having five to eight carbon atoms which may be substituted by one or more low alkyl groups; said alkyl, alkenyl or cycloalkyl groups may themselves be substituted by one or more hydroxy groups;
R₃ and/or R₄ may also represent an aryl or aralkyl group having formula: 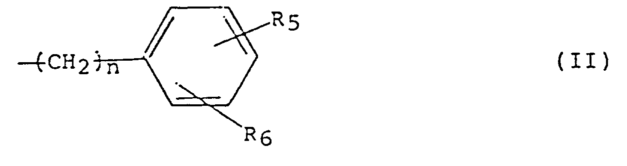 wherein:
n is between 0 and 4 and wherein R₅ and/or R₆, independently of each other, represent a hydrogen atom, a low C₁-C₆ alkyl group, a nitro group, hydroxy group or a halogen atom, or a carboxylic group, as well as salts, esters and amides thereof;
R₃ and R₄, together with the nitrogen atom to which they are bonded, may form a heterocycle having three to seven carbon atoms;
R₁ and R₂ represent a hydrogen atom or a carbamoyl group having formula: 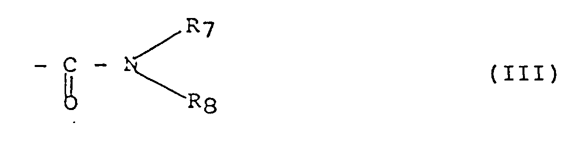 provided that one group represents hydrogen and the other represents a carbamoyl group;
R₇ and/or R₈ represent, independently of each other, a hydrogen atom, a linear or branched C₁-C₁₈ alkyl radical substituted by one or more hydroxy and/or amino groups, said alkyl chain being optionally interrupted by one or more heteroatoms selected from sulphur, nitrogen or oxygen;
R₇ and R₈ may also represent a cycloalkyl group substituted by a hydroxy, amino and/or carboxylic group;
R₇ and R₈ may form a heterocycle with the nitrogen atom to which they are bonded, the heterocycle being selected from the following groups: morpholino, piperidino, pyrrolidino, piperazino, 4'-N-alkyl piperazino, wherein the alkyl group in the 4'-position contains one to six carbon atoms, one of which may be substituted by a hydroxy or thiazolidine group, as well as their tautomeric forms having formulae:

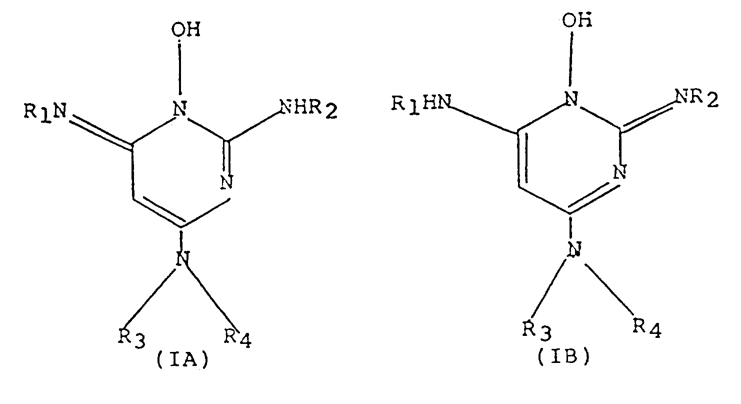

2. Compound according to claim 1, characterised in that, in formula (I) the C₁-C₁₈ alkyl radical substituted by one or more hydroxy or amino groups represented by radical R₇ or R₈, is selected from 2-hydroxy ethyl, 2,3-dihydroxy propyl, 2-hydroxy propyl, 2-hydroxy ethoxyethyl, 2-hydroxy ethylaminoethyl and 2-aminoethyl.

3. Compound according to claim 1 or claim 2, characterised in that the compound has the following formula (Ia): 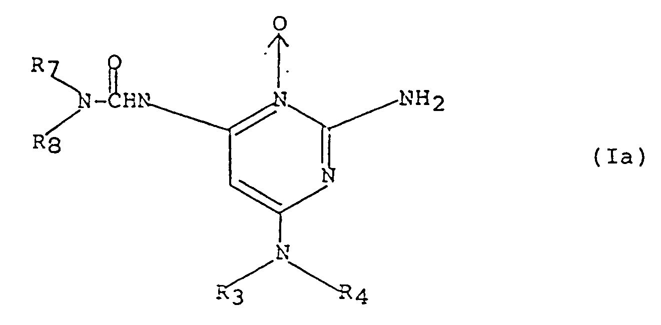 wherein R₃, R₄, R₇ and R₈ have the meanings given in claim 1.

4. Compound according to claim 1 or claim 2, characterised in that it has formulae (Ib) and (Ic): 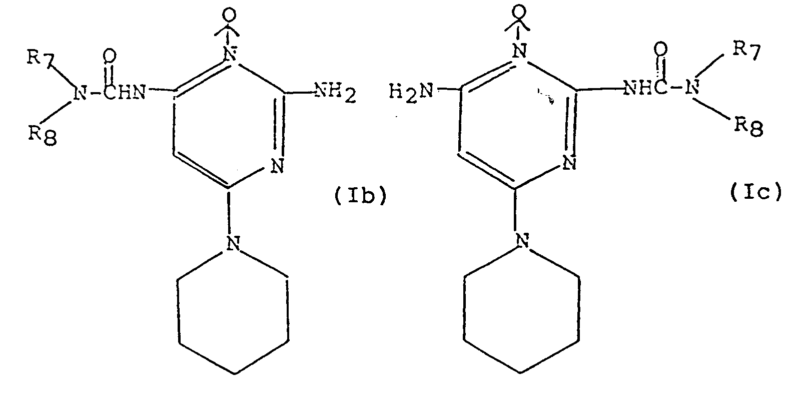 wherein R₇ and R₈ have the meanings given in claim 1.

5. Intermediate compound for the preparation of a compound in accordance with any one of claims 1 to 4, characterised in that it has the following formulae:

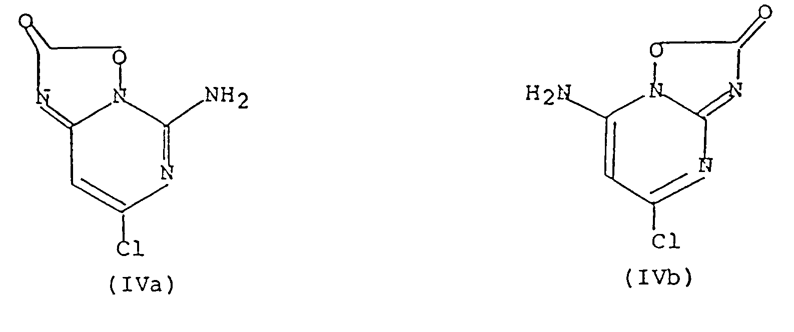

6. Method for the preparation of a compound according to any one of claims 1 to 4, characterised in that a compound according to claim 5 is treated with an amine having formula: 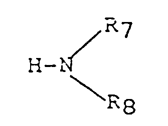 wherein R₇ and R₈ have the meanings given in claim 1,
the monourea derivative thus obtained being treated with an amine having formula: 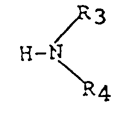 wherein R₃ and R₄ have the meanings defined in claim 1.

7. Method of preparing a compound according to claim 4, having formula (Ib): 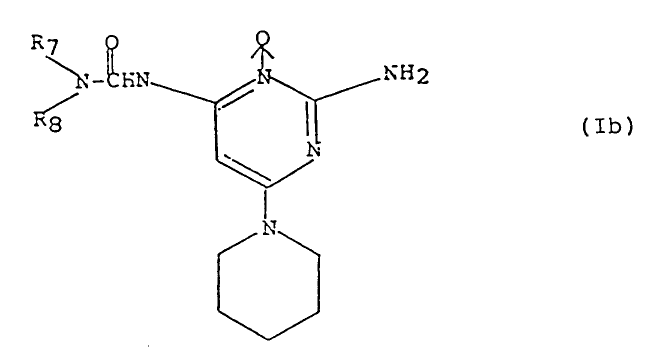 characterised in that 7-amino-5-piperidino-2H-(1,2,4)-oxadiazolo-[2,3c]-pyrimidin-2-one having formula (VIa): 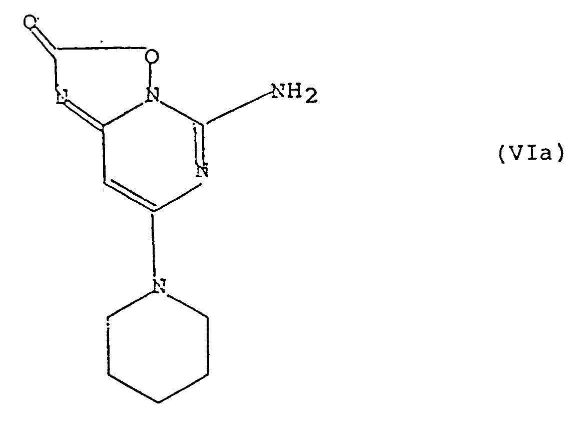 is treated with an amine having formula: 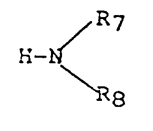 wherein R₇ and R₈ have the meanings defined in claim 1.

8. Method of preparing a compound according to claim 4, having formula (Ic): 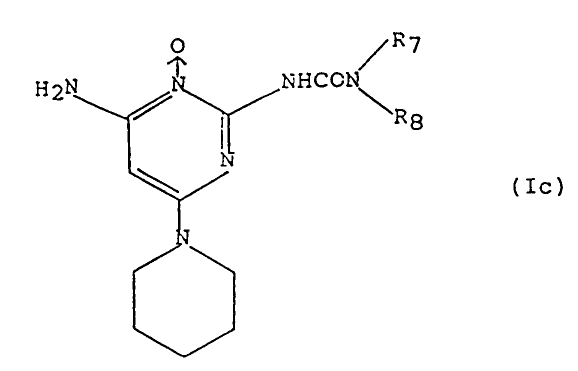 wherein R₇ or R₈ represents hydrogen, characterised in that 7-amino-5-piperidino-2H-(1,2,4)-oxadiazolo-[2,3a]-pyrimidin-2-one having formula (VIb): 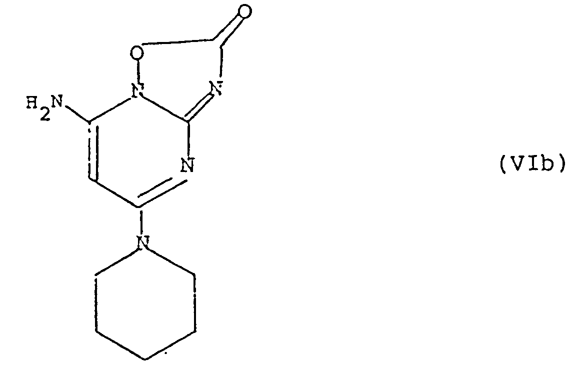 is treated with a primary amine having formula: 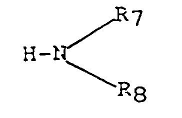 wherein R₇ and R₈ have the meanings given above.

9. Method of preparing an intermediate compound having formulae (IVa) and (IVb) in accordance with claim 5, characterised in that 2,4-diamino-6-chloro-3-oxy pyrimidine having formula (VII): 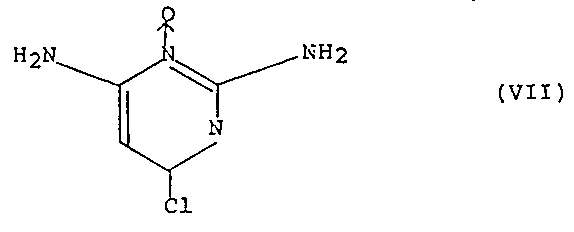 is treated in a first stage with a carbomyl chloride having formula (VIII): 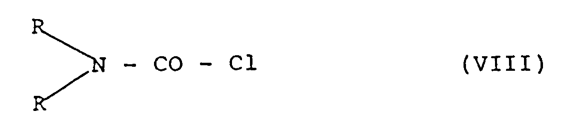 wherein R represents an alkyl group, in a polar aprotic solvent selected from dimethylsulphoxide, tetrahydrofuran or dioxane, to obtain a mixture of isomeric monourea derivatives having formulae (IXa) and (IXb): 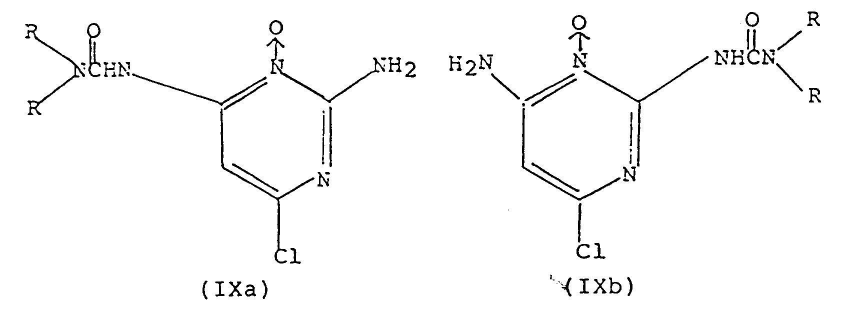 said mixture then being treated with an excess of carbamoyl chloride (formula (VIII)) to obtain 7-amino-5-chloro-2H-(1,2,4)-oxadiazolo-[2,3c]-pyrimidin-2-one of formula (IVa) and its isomer of formula (IVb).

10. A cosmetic or pharmaceutical composition characterised in that it contains, in an acceptable support for topical application, at least one compound according to any one of claims 1 to 4.

11. Pharmaceutical composition according to claim 10, characterised in that it is in the form of an unguent, tincture, cream, ointment, powder, sticking plaster, impregnated pad, solution, emulsion, lotion, gel, spray or anhydrous or aqueous suspension containing at least one compound as defined in any one of claims 1 to 4.

12. Composition according to claim 11, characterised in that the compounds having formula (I) are present in concentrations of between 0.1% and 10% by weight with respect to the total composition weight.

13. Cosmetic composition according to claim 10 in the form of a lotion, gel, soap or shampoo characterised in that it contains at least one compound according to any one of claims 1 to 4 in a concentration of between 0.01% and 5% by weight in a physiologically acceptable support.

14. Composition according to claim 13, characterised in that it further contains a hydrating agent, antiseborrheic agent, hair regrowth agent, steroidal or nonsteroidal antiflammatory agent, carotenoid, or 5,8,11,14-eicosatetraynoic acid or 5,8,11-eicosatriynoic acid and esters or amides thereof.

15. Composition according to any one of claims 10 to 14, characterised in that it also contains a preservative, stabiliser, pH regulator, osmotic pressure modifier, emulsifying agent, UV-A and UV-B filter or antioxidising agent.

16. A cosmetic hair treatment characterised in that a composition is applied to the scalp and contains at least one composition as defined in any one of claims 10 to 15.

17. Use of a compound according to any one of claims 1 to 4 for the preparation of a medicament for the treatment of alopecia, hair loss or desquamating dermatitis.

## Claims (Claims for the following Contracting State(s): ES)

1. A method for the preparation of a compound having the formula: 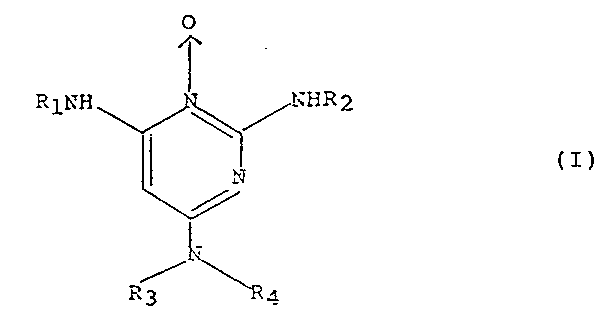 wherein:
R₃ and R₄ may be identical or different and represent a hydrogen atom, a C₁-C₁₈ linear or branched alkyl radical, an alkenyl radical having two to 18 carbon atoms, a cycloalkyl radical having five to eight carbon atoms which may be substituted by one or more low alkyl groups;
the alkyl, alkenyl or cycloalkyl groups may themselves be substituted by one or more hydroxy groups;
R₃ and/or R₄ may also represent an aryl or aralkyl group having formula: 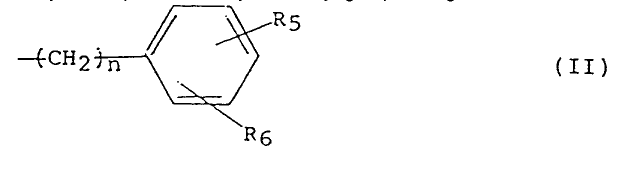 wherein:
n is between 0 and 4 and wherein R₅ and/or R₆, independently of each other, represent hydrogen, a low C₁-C₆ alkyl group, a nitro group, a hydroxy group or a halogen atom, or a carboxylic group as well as salts, esters and amides thereof;
R₃ and R₄, together with the nitrogen atom to which they are bonded, may form a heterocycle having three to seven carbon atoms;
R₁ and R₂ represent a hydrogen atom or a carbamoyl group having formula: 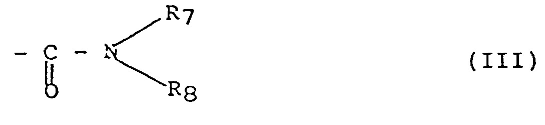 provided that one group represents hydrogen and the other represents a carbamoyl group;
R₇ and/or R₈ represent, independently of each other, a hydrogen atom, a linear or branched C₁-C₁₈ alkyl radical substituted by one or more hydroxy and/or amino groups, said alkyl chain being optionally interrupted by one or more heteroatoms selected from sulphur, nitrogen or oxygen;
R₇ and R₈ may also represent a cycloalkyl group substituted by a hydroxy, amino and/or carboxylic group;
R₇ and R₈ may form a heterocycle with the nitrogen atom to which they are bonded, the heterocycle being selected from the following groups: morpholino, piperidino, pyrrolidino, piperazino, 4'-N-alkyl piperazino, wherein the alkyl group in the 4'-position contains one to six carbon atoms, one of which may be substituted by a hydroxy or thiazolidine group, as well as their tautomeric forms having formulae: 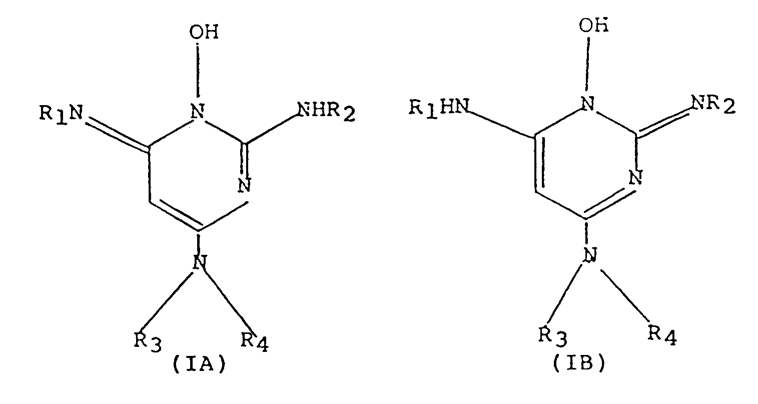 characterised in that:
(A) in order to prepare compounds having formula (IA): 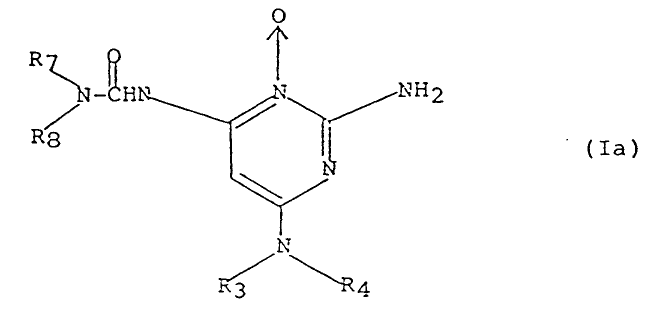 wherein R₃, R₄, R₇ and R₈ have the meanings given above or their isomers, 7-amino-5-chloro-1,2,4-oxadiazolo-[2,3c]-pyrimidin-2-one having formula (IVa) or its isomer having formula (IVb): 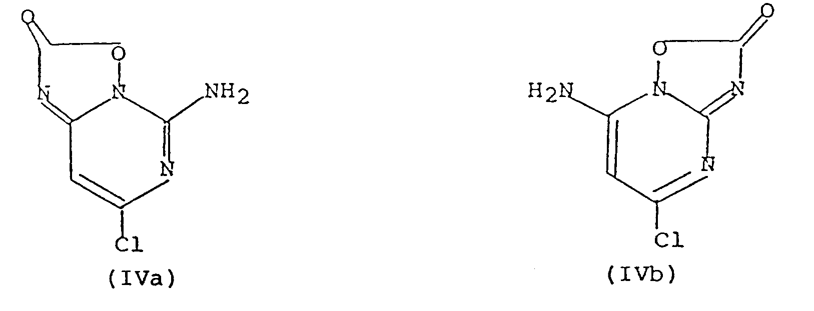 is treated in a first stage with an amine having formula: 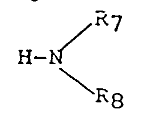 wherein R₇ and R₈ have the meanings given above, and the monourea derivative thus obtained is treated with an amine having formula: 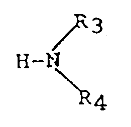 wherein R₃ and R₄ have the meanings given above;
(B) in order to prepare compounds having formula (Ib): 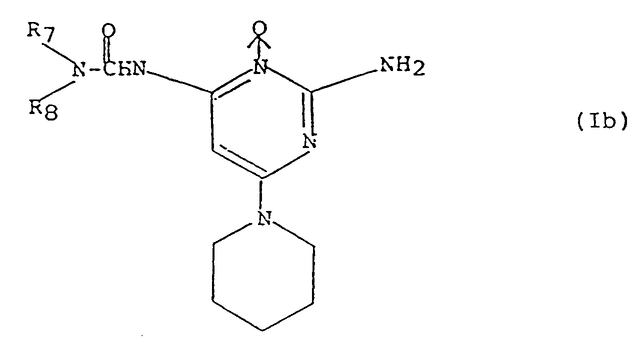 wherein R₇ and R₈ have the meanings given above, 7-amino-5-piperidino-2H-(1,2,4)-oxadiazolo-[2,3c]-pyrimidin-2-one having formula (VIa): 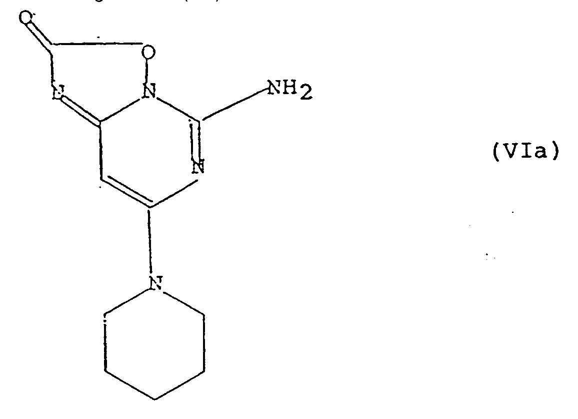 is treated with an amine having formula: 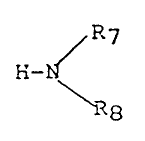
(C) in order to prepare compounds having formula (Ic): 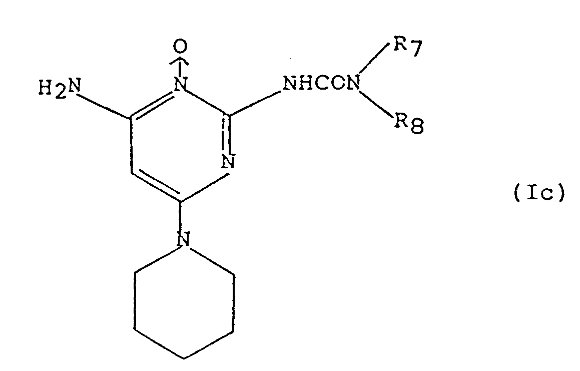 wherein R₇ and R₈ have the meanings given above, 7-amino-5-piperidino-2H-(1,2,4)-oxadiazolo-[2,3c]-pyrimidin-2-one having formula (VIb): 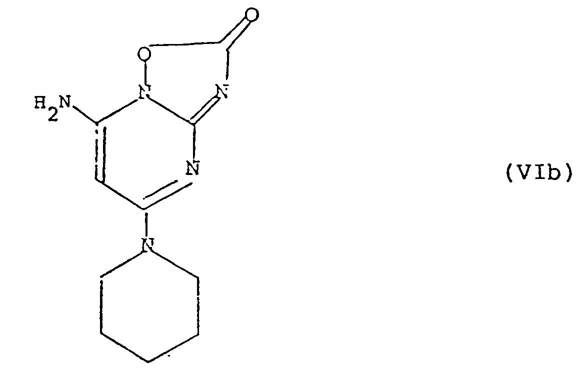 is treated with a primary amine having formula:

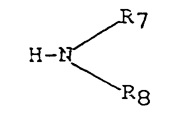

2. Method according to claim 1, characterised in that, in formula (I), the C₁-C₁₈ alkyl radical substituted by one or more hydroxy or amino groups, represented by radical R₇ or R₈, is selected from the following radicals: 2-hydroxy ethyl, 2,3-dihydroxy propyl, 2-hydroxy propyl, 2-hydroxy ethoxyethyl, 2-hydroxy ethylaminoethyl and 2-aminoethyl.

3. Method of preparation of intermediate compounds having formulae (IVa) and (IVb) as defined in claim 1, characterised in that 2,4-diamino-6-chloro-3-oxy-pyrimidine having formula (VII): 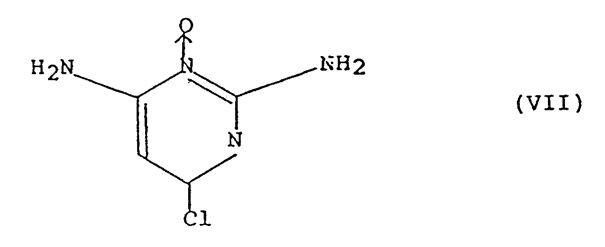 is treated in a first stage with a carbamoyl chloride having formula (VIII): 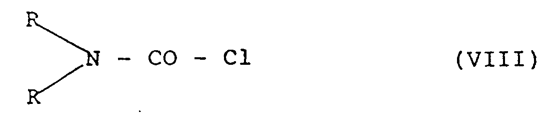 wherein R represents an alkyl group, in a polar aprotic solvent selected from dimethylsulphoxide, tetrahydrofuran or dioxane, to obtain a mixture of isomeric monourea derivatives having formulae (IXa) and (IXb): 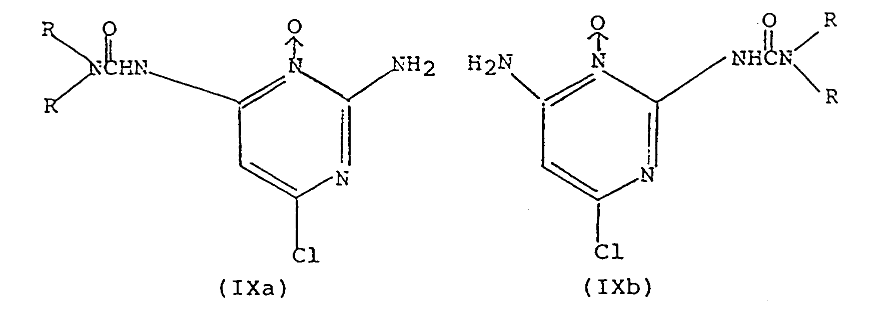 said mixture then being treated with an excess of carbamoyl chloride (formula (VIII)) to obtain 7-amino-5-chloro-2H-(1,2,4)-oxadiazolo-[2,3c]-pyrimidin-2-one having formula (IVa) and its isomer having formula (IVb).

4. Cosmetic composition in the form of a lotion, gel, soap or shampoo, characterised in that it contains, in a physiologically acceptable support which is appropriate for topical application, at least one compound as defined in claim 1 or claim 2, in a concentration between 0.01% and 5% by weight with respect to the total composition weight.

5. Composition according to claim 4, characterised in that it further contains a hydrating agent, antiseborrheic agent, hair regrowth agent, steroidal or nonsteroidal antiflammatory agent, carotenoid, 5,8,11,14-eicosatetraynoic acid or 5,8,11-eicosatriynoic acid and esters and amides thereof.

6. Composition according to claim 4 or claim 5, characterised in that it also contains a preservative, stabiliser, pH regulator, osmotic pressure modifier, emulsifying agent, UV-A and UV-B filter or antioxidising agent.

7. Method for the preparation of a pharmaceutical composition for the treatment of scalp disorders, in the form of an unguent, tincture, cream, ointment, powder, sticking plaster, impregnated pad, solution, emulsion, gel, spray, anhydrous or aqueous suspension, characterised in that at least one compound according to claim 1 or claim 2 is incorporated into a pharmaceutically acceptable support which is appropriate for topical application in concentrations between 0.1% and 100% by weight.

## Patentansprüche (Patentansprüche für folgende(n) Vertragsstaat(en): AT, BE, CH, DE, FR, GB, IT, LI, NL, SE)

1. Verbindung, **gekennzeichnet durch** die Formel 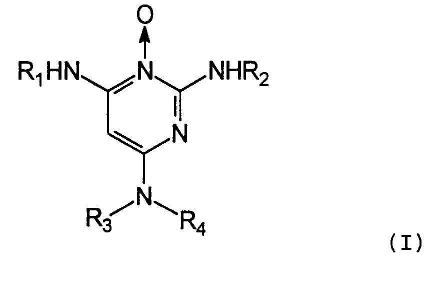 worin:
R₃ und R₄, die gleich oder verschieden sind, darstellen: ein Wasserstoffatom, eine lineare oder verzweigte C₁-C₁₈-Alkylgruppe, ein Alkenylgruppe mit 2 bis 18 Kohlenstoffatomen, ein Cycloalkylgruppe mit 5 bis 8 Kohlenstoffatomen; gegebenenfalls substituiert durch eine oder mehrere Niederalkylgruppen;
wobei die Alkyl-, Alkenyl- oder Cycloalkylgruppen selbst mit einer oder mehreren Hydroxylgruppen substituiert sein können,
R₃ und/oder R₄ gleichermaßen eine Arylgruppe oder Aralkylgruppe der Formel: 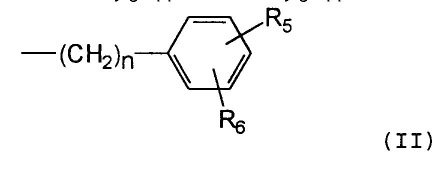 bezeichnen, worin:
n Werte von 0 bis 4 annimmt und worin R₅ und/oder R₆ unabhängig voneinander Wasserstoff, eine C₁-C₆-Alkylgruppe, eine Nitrogruppe, eine Hydroxylgruppe oder ein Halogenatom oder darüber hinaus eine Carboxylgruppe sowie Salzformen, Ester und Amide dieser letzteren darstellen,
R₃ und R₄ zusammengenommen mit dem Stickstoffatom, an das sie gebunden sind, einen Heterocyclus mit 3 bis 7 Kohlenstoffatomen bilden können,
R₁ und R₂ ein Wasserstoffatom oder eine Carbamoylgruppe der Formel: 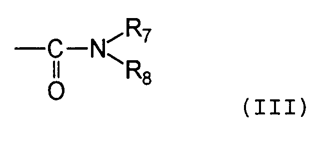 bezeichnen, mit der Maßgabe, daß eine Gruppe ein Wasserstoffatom und die andere eine Carbamoylgruppe bezeichnet;
R₇ und/oder R₈ unabhängig voneinander bezeichnen ein Wasserstoffatom, eine lineare oder verzweigte C₁-C₁₈-Alkylgruppe, die mit einer oder mehreren Hydroxyl- und/oder Aminogruppen substituiert ist, wobei diese Alkylkette gegebenenfalls mit einem oder mehreren Heteroatomen, ausgewählt aus Schwefel, Stickstoff oder Sauerstoff, unterbrochen sein kann,
R₇ und/oder R₈ ebenso eine Cycloalkylgruppe bezeichnen, die mit einer Hydroxyl-, Amino- und/oder Carboxylgruppe substituiert ist,
R₇ und R₈ mit dem Stickstoffatom, an das sie gebunden sind, einen Heterocyclus bilden können, der ausgewählt ist aus den Gruppen Morpholino, Piperidino, Pyrrolidino, Piperazino, N-Alkyl-4'-piperazino, wobei die Alkylgruppe in der 4'-Position 1 bis 6 Kohlenstoffatome enthält, von denen eines mit einer Hydroxyl- oder Thiazolidingruppe substituiert sein kann, ebenso wie ihre tautomeren Formen der Formeln:

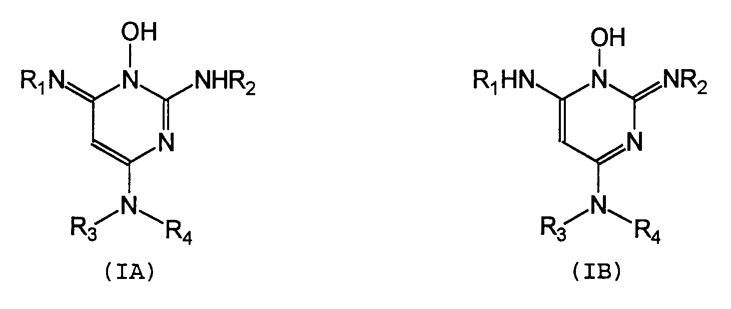

2. Verbindung gemäß Anspruch 1, dadurch **gekennzeichnet,** daß in der Formel (I) die durch die Gruppe R₇ oder R₈ dargestellte C₁-C₁₈-Alkylgruppe, die mit einer oder mehreren Hydroxyl- oder Aminogruppen substituiert ist, ausgewählt ist aus den Gruppen 2-Hydroxyethyl, 2,3-Dihydroxypropyl, 2-Hydroxypropyl, 2-Hydroxyethoxyethyl, 2-Hydroxyethylaminoethyl, 2-Aminoethyl.

3. Verbindung gemäß einem der Ansprüche 1 bis 2, dadurch **gekennzeichnet,** daß sie durch die folgende Formel (1a) dargestellt wird: 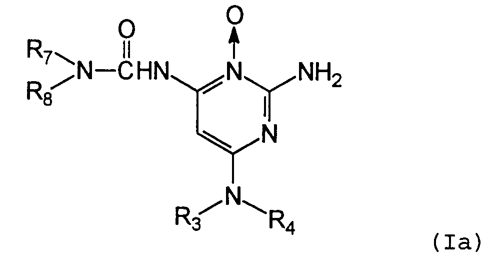 worin R₃, R₄, R₇ und R₈ die in Anspruch 1 angegebenen Bedeutungen haben.

4. Verbindung gemäß Anspruch 1 oder 2, dadurch **gekennzeichnet,** daß sie durch die Formeln (Ib) und (Ic) dargestellt wird: 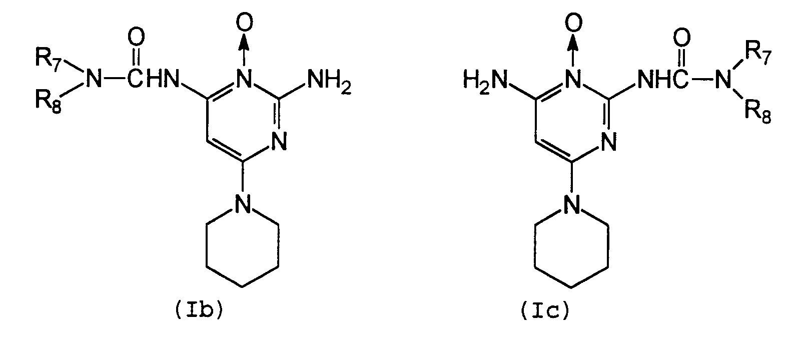 worin R₇ und R₈ die in Anspruch 1 angegebenen Bedeutungen haben.

5. Zwischenverbindung zur Herstellung von Verbindungen gemäß einem der Ansprüche 1 bis 4, dadurch **gekennzeichnet,** daß sie durch die folgenden Formeln dargestellt wird:

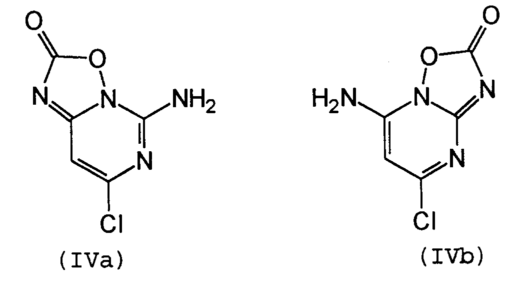

6. Verfahren zur Herstellung von Verbindungen gemäß einem der Ansprüche 1 bis 4, dadurch **gekennzeichnet,** daß man die Verbindung gemäß Anspruch 5 mit einem Amin der Formel: 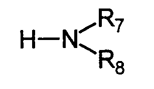 worin R₇ und R₈ die in Anspruch 1 angegebenen Bedeutungen haben, behandelt, und
daß man das resultierende Monoharnstoffderivat mit einem Amin der Formel: 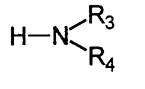 worin R₃ und R₄ die in Anspruch 1 angegebenen Bedeutungen haben, behandelt.

7. Verfahren zur Herstellung von Verbindungen gemäß Anspruch 4 der Formel (Ib): 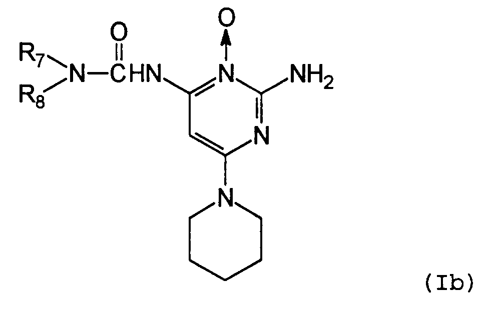 dadurch **gekennzeichnet,** daß man 7-Amino-5-piperidino-2H-1,2,4-oxadiazolo-[2,3c]-2-pyrimidinon der Formel (VIa): 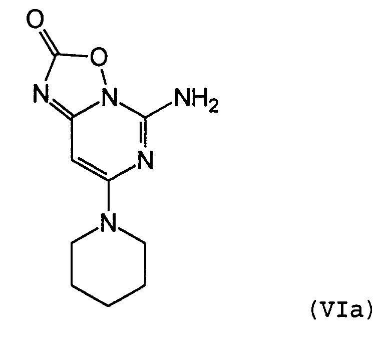 mit einem Amin der Formel 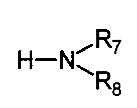 worin R₇ und R₈ die in Anspruch 1 angegebenen Bedeutungen haben, behandelt.

8. Verfahren zur Herstellung einer Verbindung gemäß Anspruch 4, dargestellt durch die Formel (Ic): 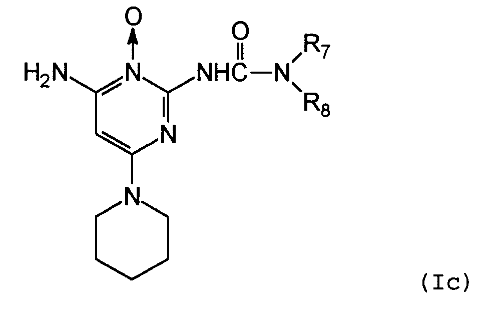 worin R₇ oder R₈ ein Wasserstoffatom darstellen, dadurch **gekennzeichnet,** daß man 7-Amino-5-piperidino-2H-1,2,4-oxadizolo-[2,3a]-2-pyrimidinon der Formel (VIb): 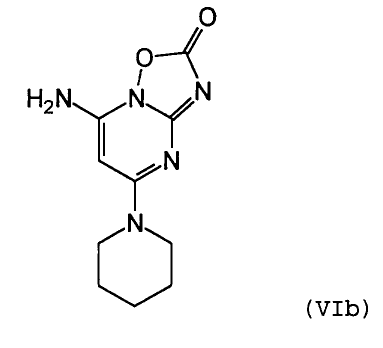 mit einem primären Amin der Formel: 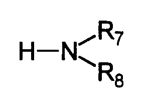 worin R₇ und R₈ die oben angegebenen Bedeutungen haben, behandelt.

9. Verfahren zur Herstellung von Zwischenverbindungen der Formeln (IVa) und (IVb) gemäß Anspruch 5, dadurch **gekennzeichnet,** daß man in einem ersten Schritt 2,4-Diamino-6-chloropyrimidin-3-oxid der Formel (VII): 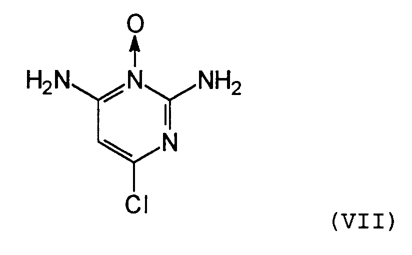 mit einem Carbamoylchlorid der Formel (VIII): 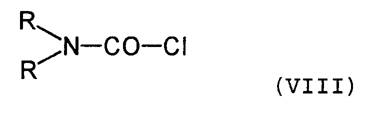 worin R eine Alkylgruppe bezeichnet, in einem polaren aprotischen Lösungsmittel behandelt, das ausgewählt ist aus Dimethylsulfoxid, Tetrahydrofuran oder Dioxan, so daß man eine Mischung aus isomeren Monoharnstoffderivaten der Formeln (IXa) und (IXb) erhält: 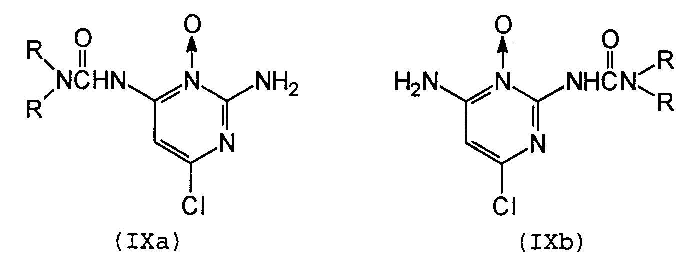 und daß man diese Mischung mit einem Überschuß Carbamoylchlorid (Formel VIII) behandelt, um 7-Amino-5-chloro-2H-1,2,4-oxadiazolo-[2,3c]-2-pyrimidinon der Formel (IVa) und sein Isomer der Formel (IVb) zu erhalten.

10. Kosmetische oder pharmazeutische Zusammensetzung, dadurch **gekennzeichnet,** daß sie in einem für eine topische Applikation geeigneten Träger mindestens eine Verbindung gemäß einem der Ansprüche 1 bis 4 enthält.

11. Pharmazeutische Zusammensetzung gemäß Anspruch 10, dadurch **gekennzeichnet,** daß sie in Form von Salben, Tinkturen, Cremes, Pomaden, Pulvern, Pflastern, getränkten Tampons, Lösungen, Emulsionen, Lotionen, Gelen, Sprays oder wasserfreien oder wäßrigen Suspensionen vorliegt, die mindestens eine Verbindung gemäß einem der Ansprüche 1 bis 4 enthalten.

12. Zusammensetzung gemäß Anspruch 11, dadurch **gekennzeichnet,** daß die Verbindungen der Formel (I) in Konzentrationen zwischen 0,1 und 10 Gew.-%, bezogen auf das Gesamtgewicht der Zusammensetzung, enthalten sind.

13. Kosmetische Zusammensetzung gemäß Anspruch 10 in Form von Lotionen, Gelen, Seifen, Shampoos, dadurch **gekennzeichnet,** daß sie in einem physiologisch akzeptablen Träger mindestens eine Verbindung gemäß einem der Ansprüche 1 bis 4 in einer Konzentration zwischen 0,01 und 5 Gew.-% enthalten.

14. Zusammensetzung gemäß Anspruch 13, dadurch **gekennzeichnet,** daß sie außerdem Hydratisierungsmittel, Antischuppenmittel, haarwuchsverstärkende Mittel, entzündungshemmende steroide oder nicht-steroide Mittel, Carotinoide, 5,8,11,14-Eicosatetrainsäure und 5,8,11-Eicosatriinsäure und deren Ester und Amide enthalten.

15. Zusammensetzung gemäß einem der Ansprüche 10 bis 14, dadurch **gekennzeichnet,** daß sie außerdem Konservierungsmittel, Stabilisierungsmittel, pH-Regulatoren, Modifikatoren für den osmotischen Druck, Emulgatoren, UV-A-und UV-B-Filter oder Antioxidanzien enthalten.

16. Verfahren zur kosmetischen Behandlung von Haaren, dadurch **gekennzeichnet,** daß man auf die Kopfhaut mindestens eine Zusammensetzung gemäß einem der Ansprüche 10 bis 15 aufbringt.

17. Verwendung von Verbindungen gemäß einem der Ansprüche 1 bis 4 zur Herstellung eines Medikaments zur Behandlung des kreisförmigen Haarausfalls, des Haarausfalls oder der schuppenförmigen Dermatitis.

## Patentansprüche (Patentansprüche für folgende(n) Vertragsstaat(en): ES)

1. Verfahren zur Herstellung einer Verbindung der Formel: 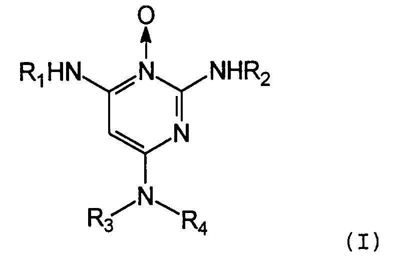 worin:
R₃ und R₄, die gleich oder verschieden sind, darstellen: ein Wasserstoffatom, eine lineare oder verzweigte C₁-C₁₈-Alkylgruppe, Alkenylgruppe mit 2 bis 18 Kohlenstoffatomen, Cycloalkylgruppe mit 5 bis 8 Kohlenstoffatomen; gegebenenfalls substituiert durch eine oder mehrere Niederalkylgruppen;
wobei die Alkyl-, Alkenyl- oder Cycloalkylgruppen selbst mit einer oder mehreren Hydroxylgruppen substituiert sein können,
R₃ und/oder R₄ gleichermaßen eine Arylgruppe oder Aralkylgruppe der Formel: 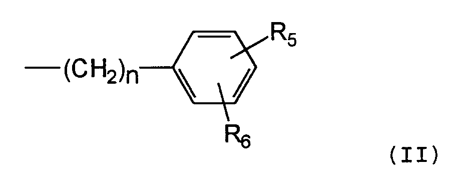 bezeichnen, worin:
n Werte von 0 bis 4 annimmt und worin R₅ und/oder R₆ unabhängig voneinander Wasserstoff, eine C₁-C₆-Alkylgruppe, eine Nitrogruppe, eine Hydroxylgruppe oder ein Halogenatom oder darüber hinaus eine Carboxylgruppe sowie Salzformen, Ester und Amide dieser letzteren Gruppen darstellen,
R₃ und R₄ zusammengenommen mit dem Stickstoffatom, an das sie gebunden sind, einen Heterocyclus mit 3 bis 7 Kohlenstoffatomen bilden können,
R₁ und R₂ ein Wasserstoffatom oder eine Carbamoylgruppe der Formel: 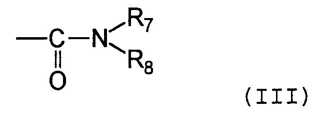 bezeichnen, mit der Maßgabe, daß eine Gruppe ein Wasserstoffatom und die andere eine Carbamoylgruppe bezeichnet;
R₇ und/oder R₈ unabhängig voneinander ein Wasserstoffatom, eine lineare oder verzweigte C₁-C₁₈-Alkylgruppe, die mit einer oder mehreren Hydroxyl- und/oder Aminogruppen substituiert ist, wobei diese Alkylkette gegebenenfalls mit einem oder mehreren Heteroatomen, ausgewählt aus Schwefel, Stickstoff oder Sauerstoff, unterbrochen sein kann,
R₇ und/oder R₈ ebenso eine Cycloalkylgruppe bezeichnen, die mit einer Hydroxyl-, Amino- und/oder Carboxylgruppe substituiert ist,
R₇ und R₈ mit dem Stickstoffatom, an das sie gebunden sind, einen Heterocyclus bilden können, der ausgewählt ist aus den Gruppen Morpholino, Piperidino, Pyrrolidino, Piperazino, N-Alkyl-4'-piperazino, wobei die Alkylgruppe in der 4'-Position 1 bis 6 Kohlenstoffatome enthält, von denen eines mit einer Hydroxyl- oder Thiazolidingruppe substituiert sein kann, ebenso wie ihrer tautomeren Formen der Formeln: 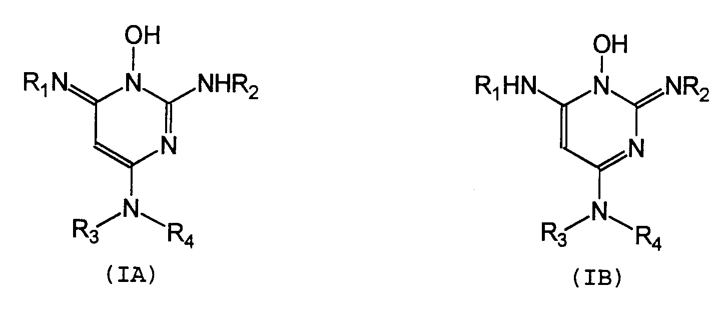 dadurch **gekennzeichnet,** daß:
(A) zur Herstellung von Verbindungen der Formel (Ia): 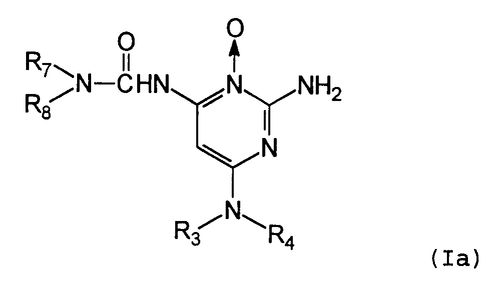 worin R₃, R₄, R₇ und R₈ die oben angegebenen Bedeutungen haben, oder von deren Isomeren, man 7-Amino-5-chloro-1,2,4-oxadiazolo-[2,3c]-2-pyrimidinon der Formel (IVa) oder sein Isomer der Formel (IVb): 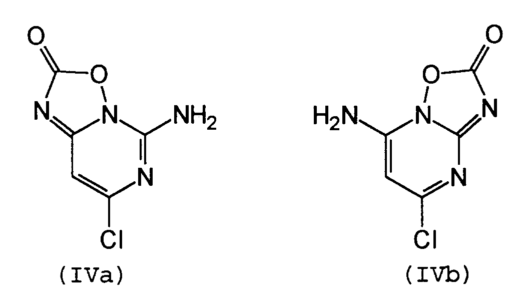 mit einem Amin der Formel: 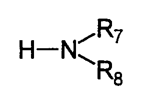 worin R₇ und R₈ die oben angegebenen Bedeutungen haben, in einem ersten Schritt behandelt und darauf das resultierende Monoharnstoffderivat mit einem Amin der Formel: 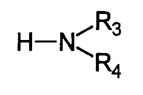 behandelt, worin R₃ und R₄ die oben angegebenen Bedeutungen haben;
(B) zur Herstellung der Verbindungen mit der Formel (Ib): 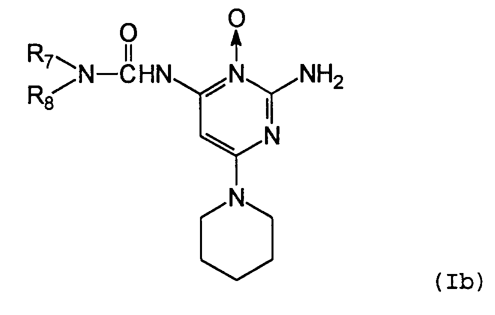 worin R₇ und R₈ die oben angegebenen Bedeutungen haben, man 7-Amino-5-piperidino-2H-1,2,4-oxadiazolo-[2,3c]-2-pyrimidinon der Formel (VIa): 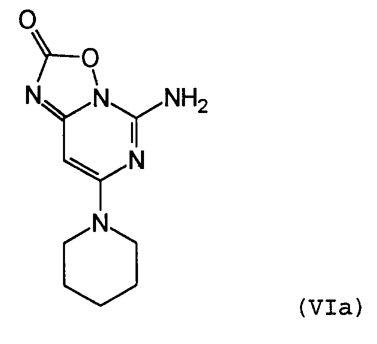 mit einem Amin der Formel: 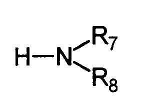 behandelt,
(C) zur Herstellung der Verbindungen mit der Formel (Ic): 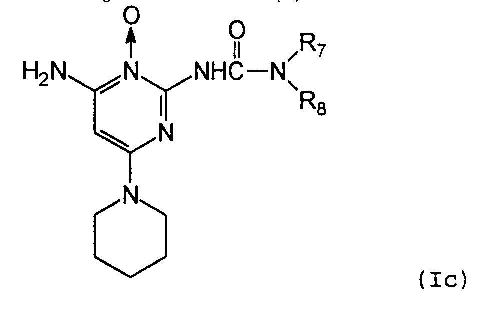 worin R₇ und R₈ die oben angegebenen Bedeutungen haben, man 7-Amino-5-piperidino-2H-1,2,4-oxadiazolo-[2,3c]-2-pyrimidinon der Formel (VIb): 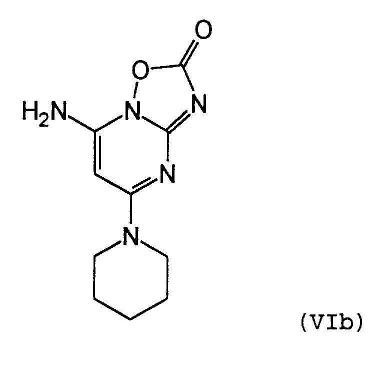 mit einem primären Amin der Formel: 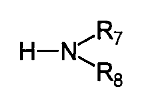 behandelt.

2. Verfahren gemäß Anspruch 1, dadurch **gekennzeichnet,** daß in Formel (I) die mit einer oder mehreren Hydroxyl- oder Aminogruppen substituierte C₁-C₁₈-Alkylgruppe, die die Gruppe R₇ oder R₈ darstellt, ausgewählt ist aus den Gruppen 2-Hydroxyethyl, 2,3-Dihydroxypropyl, 2-Hydroxypropyl, 2-Hydroxyethoxyethyl, 2-Hydroxyethylaminoethyl und 2-Aminoethyl.

3. Verfahren zur Herstellung von Zwischenprodukten der Formeln (IVa) und (IVb) gemäß Anspruch 1, dadurch **gekennzeichnet,** daß man in einem ersten Schritt 2,4-Diamino-6-chloropyrimidin-3-oxid der Formel (VII): 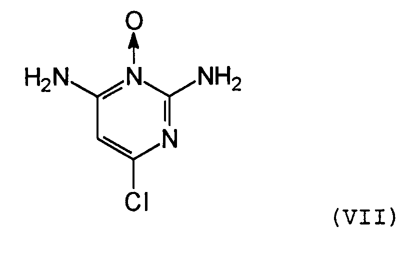 mit einem Carbamoylchlorid der Formel (VIII): 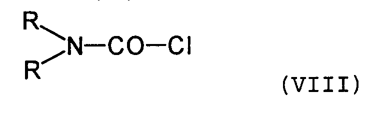 worin R eine Alkylgruppe darstellt, in einem polaren aprotischen Lösungsmittel, ausgewählt aus Dimethylsulfoxid, Tetrahydrofuran oder Dioxan, behandelt, um die Mischung der isomeren Monoharnstoffderivate der Formeln (IXa) und (IXb) zu erhalten: 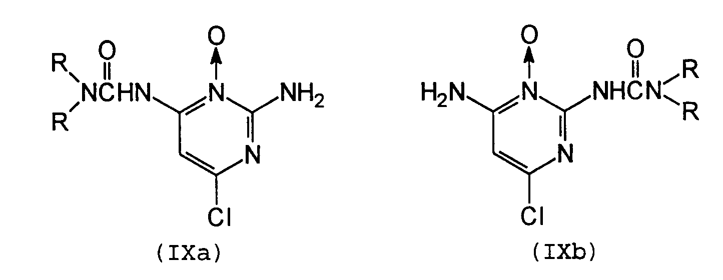 daß man diese Mischung mit einem Überschuß des Carbamoylchlorids (Formel VIII) behandelt, so daß man 7-Amino-5-chloro-2H-1,2,4-oxadiazolo-[2,3c]-2-pyrimidinon der Formel (IVa) und sein Isomer der Formel (IVb) erhält.

4. Kosmetische Zusammensetzung in Form einer Lotion, eines Gels, einer Seife, eines Shampoos, dadurch **gekennzeichnet,** daß sie in einem physiologisch annehmbaren Träger, der für eine topische Applikation geeignet ist, mindestens eine Verbindung gemäß einem der Ansprüche 1 bis 2 in einer Konzentration zwischen 0,01 und 5 Gew.-%, bezogen auf das Gesamtgewicht der Zusammensetzung, enthält.

5. Zusammensetzung gemäß Anspruch 4, dadurch **gekennzeichnet,** daß sie außerdem Hydratisierungsmittel, Antischuppenmittel, Mittel, die den Haarwuchs stärken, steroide oder nicht-steroide entzündungshemmende Mittel, Carotinoide, 5,8,11,14-Eicosatetrainsäure und 5,8,11-Eicosatriinsäure und deren Ester und Amide enthält.

6. Zusammensetzung gemäß einem der Ansprüche 4 bis 5, dadurch **gekennzeichnet,** daß sie außerdem Konservierungsstoffe, Stabilisierungsmittel, pH-regulierende Mittel, Mittel, die den osmotischen Druck modifizieren, Emulgatoren, UV-A- und UV-B-Filter, Antioxidanzien enthält.

7. Verfahren zur Herstellung einer pharmazeutischen Zusammensetzung in Form einer Salbe, einer Tinktur, einer Creme, einer Pomade, eines Pulvers, eines Pflasters, eines getränkten Tampons, einer Lösung, einer Emulsion, eines Gels, eines Sprays, einer wasserfreien oder wäßrigen Suspension, die zur Behandlung von Krankheiten der Kopfhaut bestimmt ist, dadurch **gekennzeichnet,** daß man in einem pharmazeutisch annehmbaren Träger, der zur topischen Anwendung geeignet ist, mindestens eine Verbindung gemäß Anspruch 1 oder 2 in Konzentrationen zwischen 0,1 und 10 Gew.-% inkorporiert.
